# EUROPEAN PATENT SPECIFICATION

(11) **EP 3 027 213 B1**
(45) Date of publication and mention of the grant of the patent: **18.10.2017**
(21) Application number: 14755588.2
(22) Date of filing: 27.07.2014
(51) Int. Cl.: A61K 45/06, A61K 31/165, A61K 31/19, A61K 31/5375, A61K 31/65, A61K 31/7072

(54) **COMPOSITIONS FOR AMELIORATING AND/OR PREVENTING ADVERSE SIDE EFFECTS IN ANTIBIOTIC THERAPY**
ZUSAMMENSETZUNGEN ZUR VERBESSERUNG UND/ODER VERHINDERUNG UNERWÜNSCHTER NEBENWIRKUNGEN BEI EINER ANTIBIOTIKATHERAPIE
COMPOSITIONS POUR AMÉLIORER ET/OU PRÉVENIR DES EFFETS SECONDAIRES D'ANTIBIOTHÉRAPIE

(30) Priority: 31.07.2013 EP 13003810
(43) Date of publication of application: 08.06.2016
(73) Proprietor: Mitogenomix GmbH, 97292 Uettingen (DE)
(72) Inventor: SEIBEL, Martina, 97292 Uettingen (DE)
(74) Representative: Schüssler, Andrea
(86) International application number: PCT/EP2014/002052
(87) International publication number: WO 2015/014470

(56) References cited:
- WO-A1-96/37228
- WO-A1-2013/078554
- WO-A1-2013/103780
- WO-A2-2007/044700
- US-A- 5 633 285
- MORAIS R ET AL: "On the contribution of the mitochondrial genome to the growth of Chinese hamster embryo cells in culture", CANADIAN JOURNAL OF BIOCHEMISTRY, NATIONAL RESEARCH COUNCIL OF CANADA, OTTAWA, CA, vol. 60, no. 3, 1 January 1982 (1982-01-01), pages 290-294, XP008166172, ISSN: 0008-4018
- FRIGO I V: "Some indices of nonspecific reactivity in patients with syphilis under continuous treatment with antibiotics with methyluracil (Russian)", EMBASE,, 1 January 1974 (1974-01-01), XP002717540,
- Jian Liu ET AL: "Hypoxia and pyruvate/uridine have synergic effect on induction of stemness factors in human esophageal cancer cells", Life Science Journal, 1 January 2012 (2012-01-01), XP055159720, Retrieved from the Internet: URL:http://www.lifesciencesite.com/lsj/lif e0903/045_9782life0903_326_334.pdf [retrieved on 2014-12-19]
- DATABASE EMBASE [Online] ELSEVIER SCIENCE PUBLISHERS, AMSTERDAM, NL; 1974, ABDOU M A F: "Synthetic medium for testing of susceptibility. I. Susceptibility of bacteria to antibiotics and chemotherapeutic agents", XP002734064, Database accession no. EMB-1975167944 & ABDOU M A F: "Synthetic medium for testing of susceptibility. I. Susceptibility of bacteria to antibiotics and chemotherapeutic agents", ZBL.BAKT.REIHE A 1974, vol. 229, no. 2, 1974, pages 216-231,

## Description

### Area of the Invention

The present invention relates to a pharmaceutical composition comprising: (i) a therapeutically effective amount of at least one antibiotic; and (ii) a therapeutically effective amount of an uridine compound and a pyruvate compound. The formulation is prepared in a suitable dosage form for the delivery of antibiotics and the reduction of the known adverse side-effects of the antibiotics is achieved by the added uridine and/or pyruvate compounds when administered to a subject. The present invention neutralizes highly efficient these side-effects without interfering with the antibiotic effect of the drugs. By co-administration of (i) antibiotics and (ii) uridine and pyruvate compounds, cell's capability to generate energy and synthesize nucleic acids is being supported. Especially side-effects caused by antibiotics that interfere with the cellular and mitochondrial metabolism are effectively removed as secondary actions of the drugs.

Also described are methods of reducing or preventing a failure of treatment for an antibiotic-treatable infection in a patient comprising administering the formulation in a suitable way of administration.

### Background of the Invention

In 1947, the term "antibiotics" was used to describe agents that are antagonistic to the growth of microorganisms in high dilution (1). Today, the term "antibiotics" has a broader appearance as it encompasses very powerful drugs that fight bacterial infections by killing bacteria or prevent their reproduction. Antibiotics are often identified as low-molecular metabolic substances produced by bacteria, fungi or higher plants. They can be isolated from these sources or are partially or fully synthetic compounds. Substances that are still isolated from living organisms belong to the group of aminoglycosides. Others (e.g. sulfonamides, quinolones or oxazolidinones) are fully synthesized. In general, there are two ways of classifying antimicrobial agents: They can be classified either by the basis of their chemical or biosynthetic origin (natural, seminatural, synthetic) or distinguished based on their biological effect on microorganisms. The latter classification discriminates between bactericidal agents that kill bacteria and bacteriostatic compounds that stall or slow down bacterial growth.

WO 96/37228 A1 and US Patent 5,633,285 concern an antibacterial-wound healing composition and methods for preparing and using same.

WO 2013/078554 A1 describes compositions of tigecycline and uses thereof.

WO 2013/103780 A1 describes compositions and methods comprising reactive oxygen species (ROS) target modulators that increase ROS flux and endogenous ROS production, thereby potentiating oxidative attack by antibiotics and biocides.

Morais et al. (Canadian J. of Biochemistry, National Research Council of Canada, Vol. 60, No. 3, pp. 290-294 (1982)), Liu et al. (Life Science Journal, page 327, 2012) and Abdou et al. (Zbl. Bakt. Reihe A 1974, Vol. 229, No. 2, pp. 216-231 (1974)) describe cell culture media.

Depending on their way or place of action, antibiotics can be divided in the following subgroups:
- cell wall construction (e.g. ß-lactams)
- structure and function of cell membrane (e.g. polymyxin B)
- folic acid synthesis (e.g. sulfonamides)
- protein synthesis (e.g. aminoglycosides)
- structure and function of DNA (e.q. quinolones)
- others (e.g. rifampin)

As all therapeutic drugs to be administered to humans or mammals require extensive testing, new compounds are screened for any negative side-effect before approval for clinical use is given (e.g. FDA). Although most approved antibiotics are considered safe and are often well tolerated by patients, some antibiotics have been associated with a range of adverse effects (2) that can range from fever and nausea to major allergic reactions, including photodermatidis and anaphylaxis. Common side effects include diarrhea and overgrowth of pathogenic bacteria and yeast species. Dramatic side effects, as for instance variations in the hemogram of patients, liver malfunction, reversible and irreversible damage of the bone marrow as well as some forms of leukemia (rare cases) have been reported. As a consequence, compounds with severe side effects experience a far less utilization and are considered as "last-line defense".

Patient's treatment has been further complicated in the last years by the emerging of multidrug resistance microbes (e.g. MRSA, Methicillin Resistant Staphylococcus Aureus). Multidrug resistance is a condition enabling disease causing microorganisms to escape distinct antibiotic, antifungal or antiviral drugs. In this context, even antibiotics previously classified exhibiting severe side-effects have gained major attention again (3). For example, the phenylpropanoid chloramphenicol is a very effective broad-spectrum.antibiotic that functions very well on anaerobic bacteria, gram-positive as well as on gram-negative bacteria. The compound has been used to treat acne, eye infections and for therapy of patients in grave cases of certain meningitis agents, tetracycline-resistant cholera, typhoid, typhus, dysentery, diphtheria, malaria. However, severe side-effects - even leading to death - were reported that degraded this antibiotic compound to the class of "last-line defenders", e.g.:
The *Gray (Grey) Baby Syndrome* is a rare but serious side-effect (4). It occurs when neonates are treated intravenously with chloramphenicol. Symptoms observed range from an ashen gray color of the skin, vomiting, limp body tone, hypotension, cyanosis, hypothermia to cardiovascular collapse. Due to a yet unknown mechanism, it is thought that a lack of glucuronidation reactions occurring in the baby leads to an accumulation of toxic metabolites (5).

Chloramphenicol can also induce a damage of bone marrow tissue that in turn can lead to severe forms of anemia (either reversible or aplastic anemia). As a consequence thereof, a malfunctioning erythropoiesis is frequently seen (6). In very rare cases, a co-incidence of chloramphenicol treatment and leukemia in childhood has been described (7).

The side-effects of antibiotics are frequently caused by interference with cell's metabolism or protein synthesis in mammalia. The latter is particular important to mitochondria as the ribosomes in these organelles are evolutionary closely linked to bacterial ribosomes. Consequently, a drug directed to interfere with bacterial protein synthesis will impede mitochondrial protein synthesis and thus will lead to dramatic effects on the overall metabolism. Mitochondria have been identified to house a variety of essential metabolic pathways. Among those, the generation of energy through the mitochondrial oxidative phosphorylation system plays a pivotal role to sustain cell's energy demand. However, to keep up the energy requirements under drugs that intercept the protein synthesis, cell's metabolism will be shifted towards the far less effective glycolytic pathway, thereby producing lactate. Thus, energy synthesis becomes the rate limiting step and is involved in the experienced side-effects of the antibiotics (e.g. chloramphenicol, linezolid, tigezycline etc.). Additionally, the diminishing number of OXPHOS complexes stall the uridine biosynthesis pathway so that the cell cycle of fast dividing cells can be arrested.

### Object

The object of the present invention is to provide compositions for ameliorating and/or preventing adverse side effects in antibiotic therapy.

### Brief Description of the Present Invention

This object is solved by the subject-matter of present independent claims 1 and 6. Preferred embodiments are mentioned in the dependent claims.

The invention provides a composition comprising:
(i) a therapeutically effective amount of at least one antibiotic; and
(ii) a therapeutically effective amount of both an uridine compound and pyruvate compound
for use as a medicament, wherein
the uridine compound is uridine, uracil, uridine phosphate(s) (e.g. uridine-5' monophosphate (UMP), uridine-5' diphosphate (UDP), uridine-5' triphosphate (UTP)) or salts thereof, UDP-glucose, cytidine-5' monophosphate (CMP), cytidine-5' diphosphate (CDP), CDP-glucose, orotic acid, orotidylate, acylated uridine or cytidine-diphosphocholine; and
the pyruvate compound is pyruvic acid or a salt thereof (e.g. methyl pyruvate, ethyl pyruvate or sodium salt of pyruvate), creatine pyruvate, phosphoenolpyruvate, 2-phosphoglycerate, 3-phosphoglycerate, 1,3-biphosphoglycerate, glycerinaldehyde 3-phosphate, dihydroxyacetone phosphate, alanine, serine, cystein, glycine, tryptophan, asparagine, aspartate, methionine, malate or oxalacetate,

The invention further provides a pharmaceutical composition comprising
(i) a therapeutically effective amount of at least one antibiotic; and
(ii) a therapeutically effective amount of both an uridine compound and a pyruvate compound
for use in preventing or minimizing adverse side effects in the antibiotic therapy of bacterial infections in a subject, wherein
the uridine compound is uridine, uracil, uridine phosphate(s) (e.g. uridine-5' monophosphate (UMP), uridine-5' diphosphate (UDP), uridine-5' triphosphate (UTP)) or salts thereof, UDP-glucose, cytidine-5' monophosphate (CMP), cytidine-5' diphosphate (CDP), CDP-glucose, orotic acid, orotidylate, acylated uridin or cytidine-diphosphocholine; and
the pyruvate compound is pyruvic acid or a salt thereof (e.g. methyl pyruvate, ethyl pyruvate or sodium salt of pyruvate), creatine pyruvate, phosphoenolpyruvate, 2-phosphoglycerate, 3-phosphoglycerate, 1,3-biphosphoglycerate, glycerinaldehyde 3-phosphate, dihydroxyacetone phosphate, alanine, serine, cystein, glycine, tryptophan, asparagine, aspartate, methionine, malate or oxalacetate.

The inventors have found out that side-effects of antibiotics that interfere with mitochochondrial protein synthesis cause a reduction of the energy generating system (enzymes of the oxidative phosphorylation system, OXPHOS). Fast dividing cells are mostly affected, as they cannot keep the number of OXPHOS complexes constant in the daughter cells. Thus, it is known from other studies that enzymes of OXPHOS are diluted as they cannot be re-synthesized under drug influence. Within 4-5 days, the number of OXPHOS complexes is under the level needed to sustain cells energy demand (approximately 8-12 %) so that a biochemical pathway shift to glycolysis will occur. In contrast, cells in resting state are less affected as they display turnover rates (half-life) of mitochondrially synthesized proteins of 8-20 days. Thus, they can cope with the blocked biosynthesis of OXPHOS so that even after a 30 days treatment, symptoms cannot be detected within this time span.

Hence, side effects developing in patients treated with antibiotic drugs are caused by two distinct ways:
- Firstly, due to the inhibition of mitochondrial protein synthesis, these antibiotics shift the energy generating pathway towards glycolysis. Thus, glycolysis needs to cover almost the entire energy demand of cells and needs to function at its best.
- Secondly, due to the diminished rate of OXPHOS complexes, a second problem, distinct from the energy underrun, kicks in: electrons from the dihydroorotate dehydrogenase (DHODH) are fed exclusively into the OXPHOS via the mitochondrial ubiquinol/ubiquinone cycle. If OXPHOS enzymes become rate limiting, the DHODH pathway comes to a halt as electrons cannot be released from the DHODH-redox reaction. The enzyme (DHODH) is linked to the essential uridine biosynthesis pathway. Thus, shortening in cell's uridine supply causes consequently a stalling of the cell cycle.

When metabolism is shifted towards anaerobic glycolysis under antibiotic treatment, cells produce lactate from pyruvate that is released into the blood. As a consequence of the occurring metabolic switch, the NAD⁺/NADH ratio is shifted towards NADH, because it cannot be re-oxidized by the few remaining OXPHOS complexes. This situation leads to a shortening of NAD⁺ and pyruvate within the cell so that essential biochemical pathways are blocked. When pyruvate is administered, both problems are solved:
- Firstly, the inner cellular pyruvate pool increases so that stalled biochemical reactions can be restarted again
- Secondly, as the NADH level can be reduced by production of lactate from the administered pyruvate, reactions previously stalled by the high NADH level can be restarted again.

Cell's metabolism is additionally challenged by the diminished uridine pool that is caused by the stalled DHODH reaction. It is known that cells uridine pool is filled by three distinct pathways:
- Firstly, the cell is fed from the salvage pathway. That is: all uridines (pyrimidines) utilized within the cell (e.g. the building blocks all nucleic acids or UDP release from UDP-glucuronic acid) are reused upon breakdown. The pyrimidine level resulting from the recycling is sufficient to sustain cell's demand in a resting cell.
- Secondly, the pyrimidine hunger of dividing cells can be saturated from the freshly synthesized building blocks via the DHODH pathway.
- Thirdly, pyrimidines can be taken up by the cell. Thus extracellular sources can additionally bring in the required building blocks for cells in mitosis.

When uridine (pyrimidine) is administered in addition to the antibiotic, cell's uridine pool is refilled. Thus, biochemical reactions diminished by the antibiotic side-effect requiring UDP (e. g. glucoronidation or nucleic acid synthesis) are bolstered up so that reactions are restarted or accelerated to a normal speed. Thus, administration of both of pyruvate and uridine ameliorates and/or prevents all symptoms seen in antibiotic treated patients (e.g. gray baby syndrome; deteriorated blood building system due to energy and/or mitotic arrest of blood cells; failure of glucuronidation reaction).

### Brief Description of the Figures:

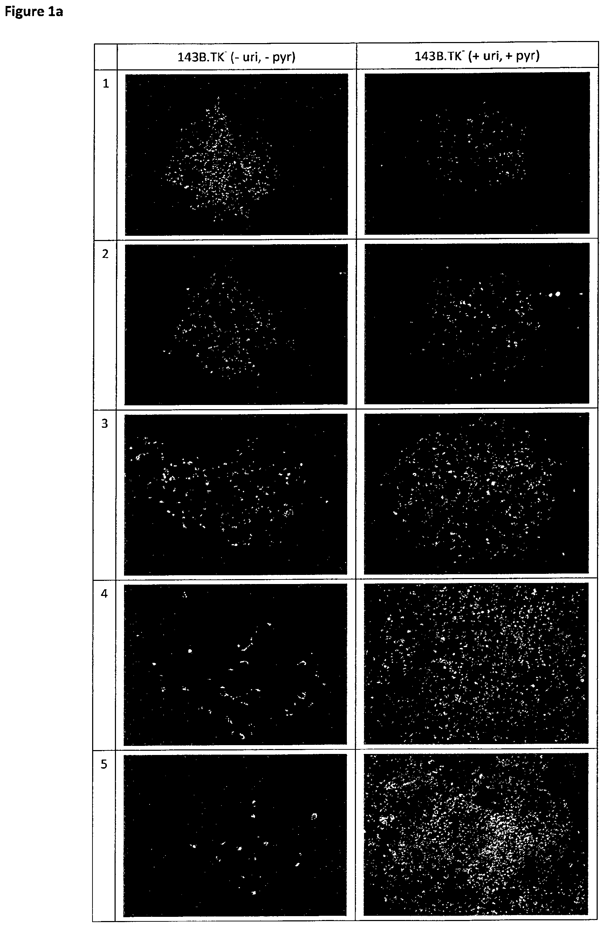

### Figure 1a: Side effect of chloramphenicol (or linezolid or tigezycline) on dividing human cells

The panel displays cell growth of a green colorized human osteosarcoma cell line (143B.TK⁻) by fluorescence microscopy. ***Left panel:*** 143B.TK⁻ cells containing a green colorizing gene (GFP, green color) were seeded and grown to the original density in normal growth medium (left, 1). At day 1, medium was changed to allow cell growth under pyruvate and uridine free conditions; the medium was supplemented with chloramphenicol (or linezolid, or tigezycline; see example 1). At day 4 (image 2), cell growths retarded and comes to a halt. At day 6 (image 3), the imaged colony starts to disperse and diminishes. At day 8 (image 4), cell number further declines and cells ceased to exist (day 10). ***Right panel:*** 143B.TK⁻ cells containing a green colorizing gene (GFP, green color) were seeded and grown to the original density in normal growth medium (right, 1). At day 1, medium was changed to allow cell growth under controlled pyruvate and uridine supply; the medium was supplemented with chloramphenicol (or linezolid, or tigezycline; see example 1). It turned out, that growth behavior of the cells was not altered by the antibiotic as the colony expanded regularly to day 10.

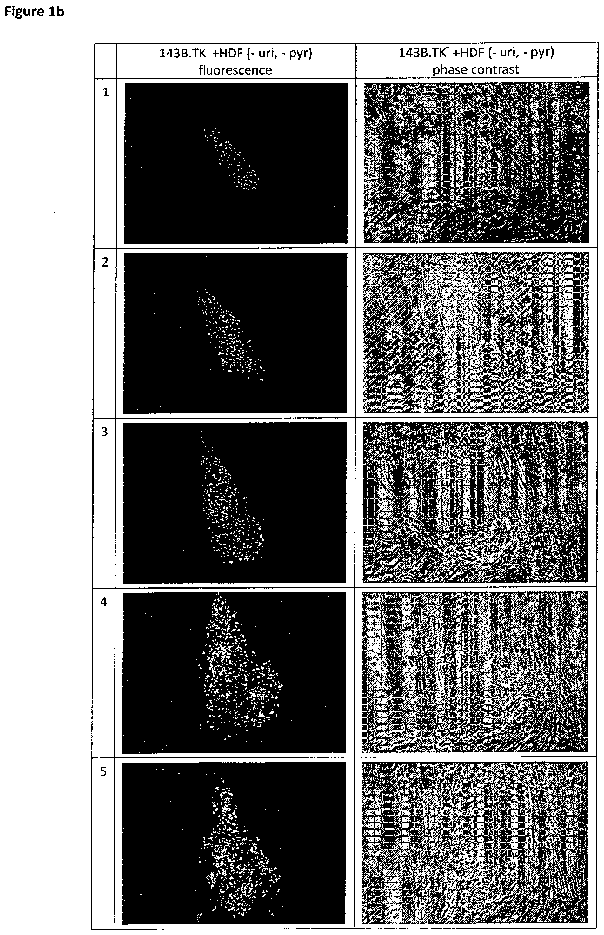

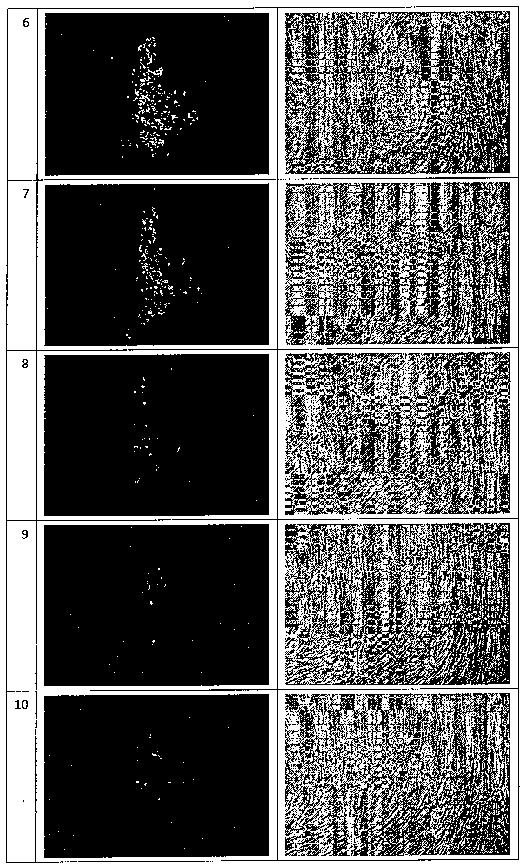

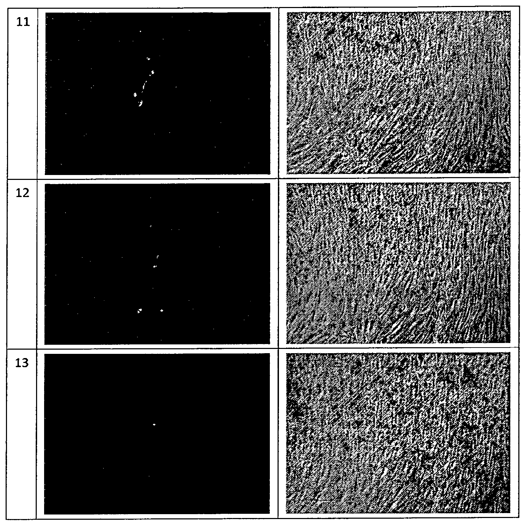

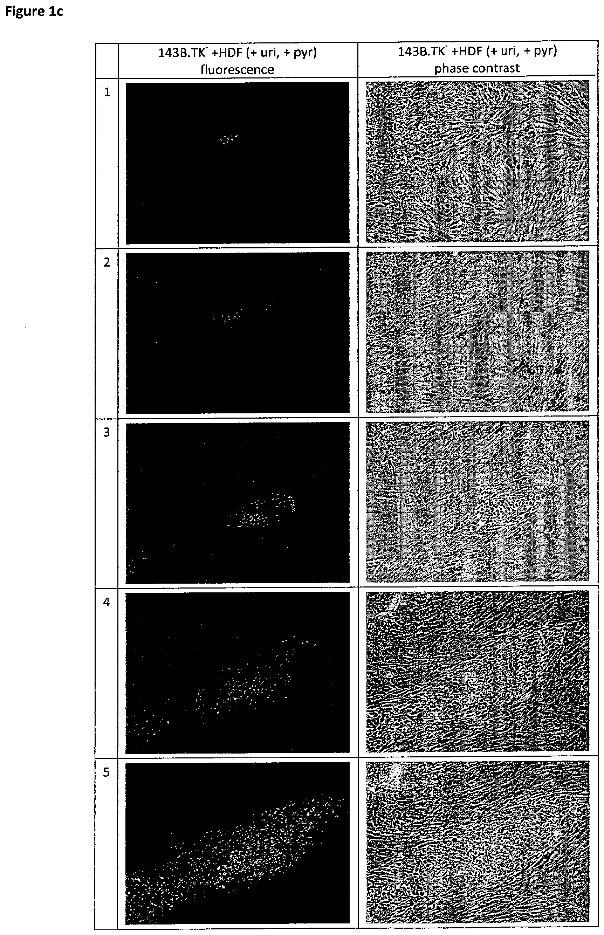

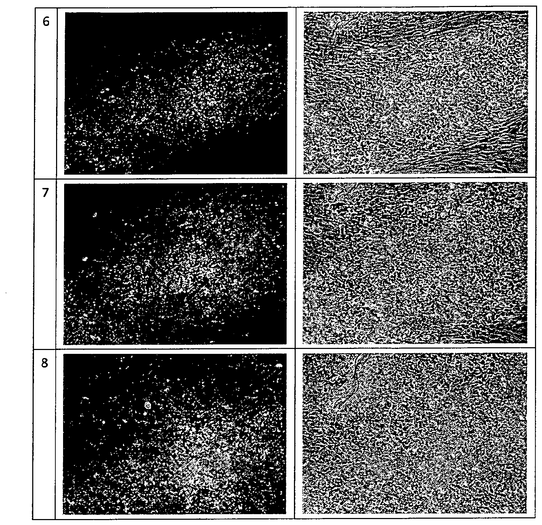

### Figure 1b and 1c: Influence of antibiotic on cell cycle in the absence or presence of pyruvate and uridine.

The panels display co-cultures grown on petri-dishes that contain a human dermal fibroblast (HDF) monolayer inoculated with GFP-colorized 143B.TK- cells imaged by fluorescence microscopy and phase contrast microscopy. The HDF cells represent a resting post-mitotic tissue, while the GFP-colorized 143B.TK- cells mimic mitotic active cells.

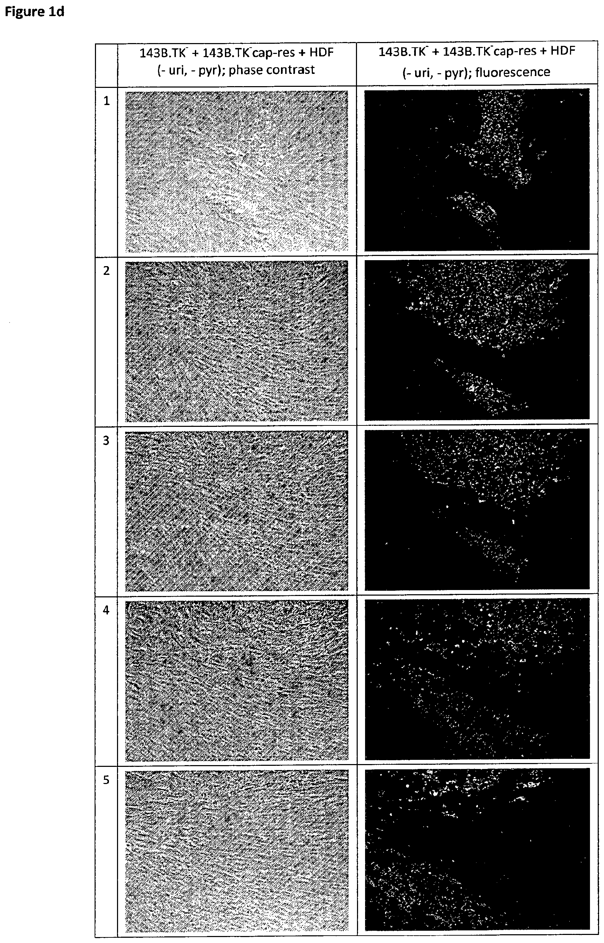

**Figure 1b****, left panel:** Without pyruvate and uridine, the side effect of chloramphenicol (or linezolid, or tigezycline) can be visualized on co-cultures similarly to the effect seen in figure 1. Approximately at day 4 of culture, the mitotic active colony enlarges and experiences growth retardation. At day 6 dispersion of the colony starts and finally ceases to exist at day 13. **Right panel:** Same area as seen in the left panel. There is no change in cell vitality seen on the HDF monolayer. Thus, the side-effect is primarily seen in mitotic active cells.
**Figure 1c****, left panel:** Co-cultured cells in the presence of pyruvate and uridine do not display any side-effect induced by chloramphenicol (or linezolid or tigezycline). The mitotic cells grow completely regularly. At day 8, the entire area is overgrown by the GFP-colorized 143B.TK⁻ cells. **Right panel:** Phase-contrast images of the same area as seen in the left panel. There is no change in cell vitality seen on the HDF monolayer mimicking post-mitotic or resting cells for more than 30 days.

### Figure 1d: Co-cultures of human dermal fibroblasts and GFP-colorized 143B.TK⁻ and with dsRED-colorized 143B.TK⁻CAP-res cells in the presence of chloramphenicol and absence of uridine and pyruvate.

The panels display co-cultures grown on petri-dishes that contain a human dermal fibroblast (HDF) monolayer inoculated with GFP-colorized 143B.TK⁻ cells and dsRED-colorized 143B.TK⁻CAP-res cells. The dsRED-colorized 143B.TK⁻CAP-res cells contain a mitochondrial genotype that renders the cells chloramphenicol resistant. Images were taken by phase contrast (left panel) and fluorescence microscopy (right panel).
**Left panel:** Phase contrast image of same area as seen in the right panel. There is no change in cell vitality seen on the HDF monolayer mimicking post-mitotic or resting cells.
**Right panel:** Colonies of approximately similar sizes were chosen (green and red). As can be seen in the images taken on subsequent days, the mitotic active cells (green color code) diminishes upon chloramphenicol side-effects when uridine and pyruvate are not supplied, whereas the chloramphenicol resistant cell line is not influenced by the side-effect of chloramphenicol and grew regularly.

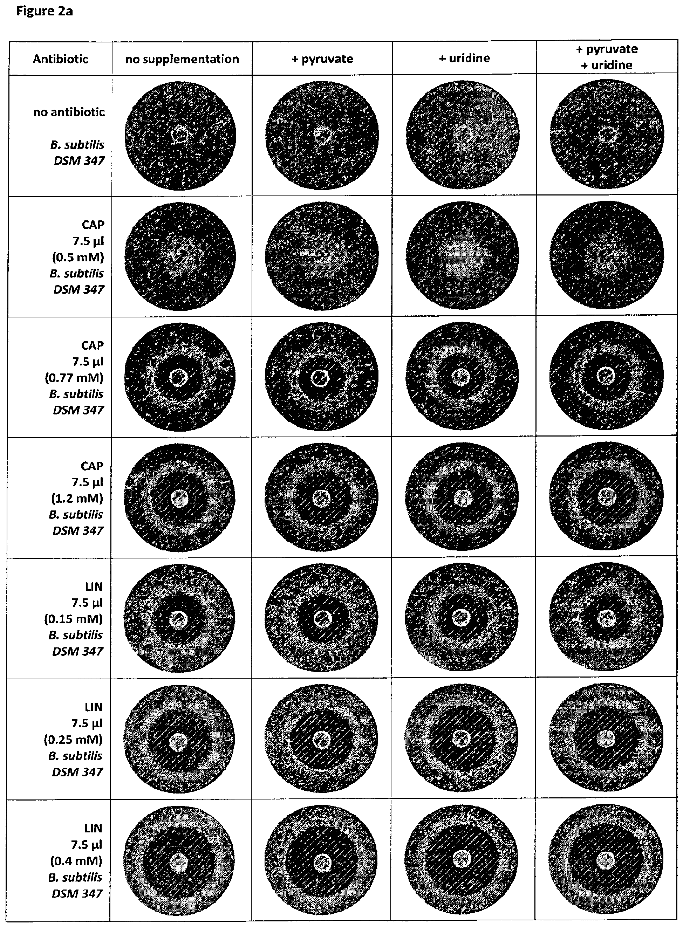

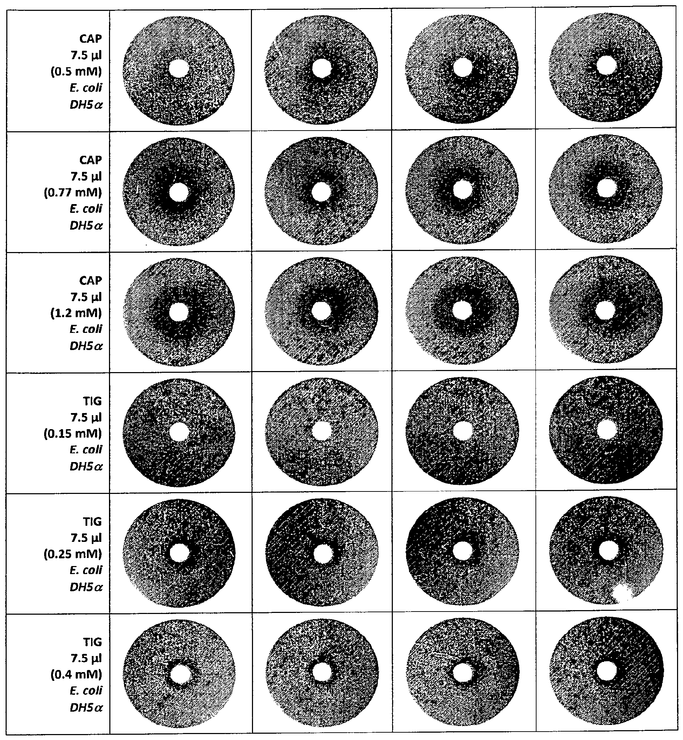

### Figure 2a: Antibiotic effect is not altered by co-administration of pyruvate, uridine or pyruvate + urine.

The panel displays the non-growth zone of bacteria (*B*. *subtilis* DSM 347 or *E. coli* DH5α) that is caused by antibiotic diffusion from the soaked paper-pads into the growth plate. The size of the non-growth zone is a measure for antibiotic activity.

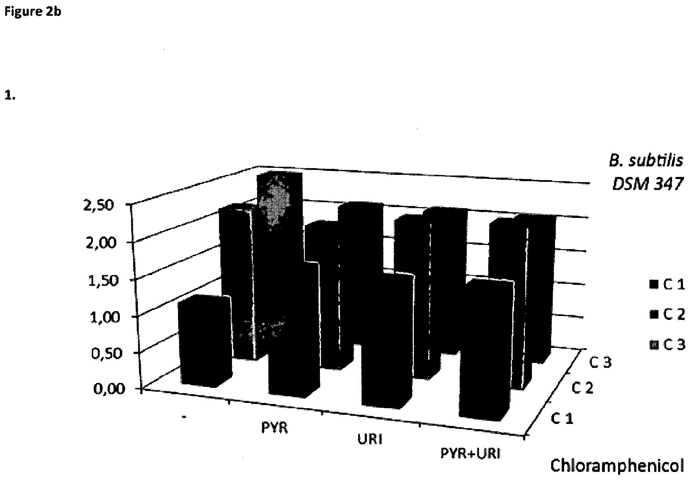

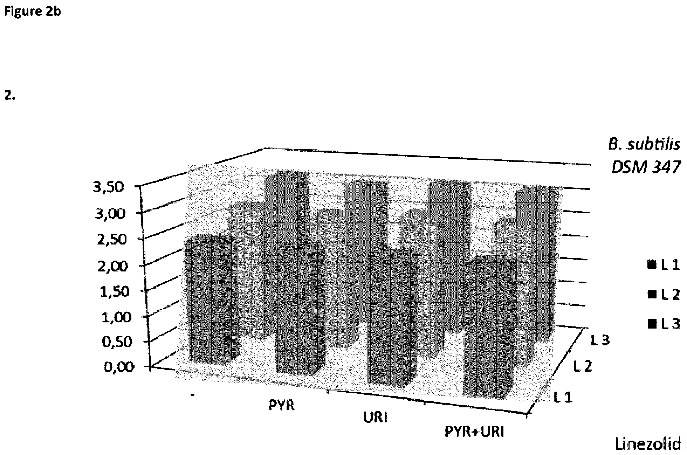

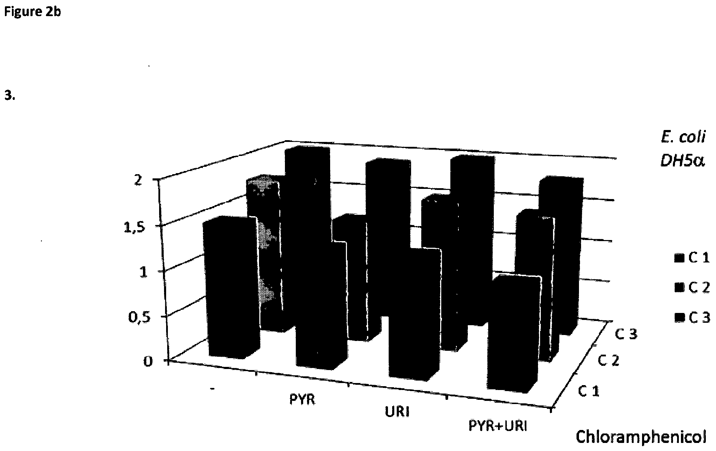

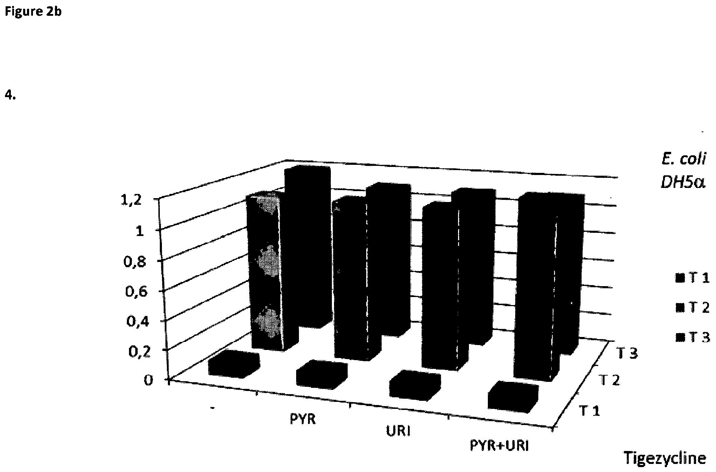

### Figure 2b: Quantitative analysis of the unaltered antibiotic effect during co-administration of pyruvate, uridine or pyruvate + uridine.

**First panel:** Antibiotic effect of chloramphenicol (C1: 0.5 mM CAP, C2: 0.77 mM CAP, C3: 1.2 mM CAP) on *B. subtilis* DSM 347. Antibiotic effect increases with concentration of antibiotic, but is independent of pyruvate, uridine or pyruvate + uridine supplementation.
**Second panel:** Antibiotic effect of linezolid (L1: 0.15 mM LIN, L2: 0.25 mM LIN, L3: 0.4 mM LIN) on *B. subtilis* DSM 347. Antibiotic effect increases with concentration of antibiotic, but is independent of pyruvate, uridine or pyruvate + uridine supplementation.
**Third panel:** Antibiotic effect of chloramphenicol (C1: 0.5 mM CAP, C2: 0.77 mM CAP, C3: 1.2 mM CAP) on *E. coli* DH5□. Antibiotic effect increases with concentration of antibiotic, but is independent of pyruvate, uridine or pyruvate + uridine supplementation.
**Fourth panel:** Antibiotic effect of tigezycline (T1: 0.15 mM TIG, T2: 0.25 mM TIG, T3: 0.4 mM TIG) on *E*. *coli* DH5α. Antibiotic effect increases with concentration of antibiotic, but is independent of pyruvate, uridine or pyruvate + uridine supplementation.

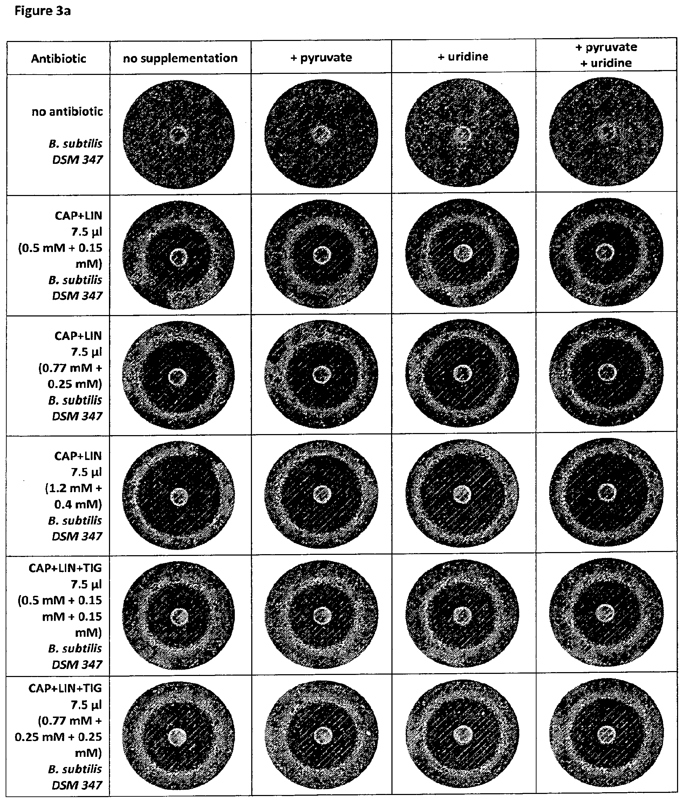

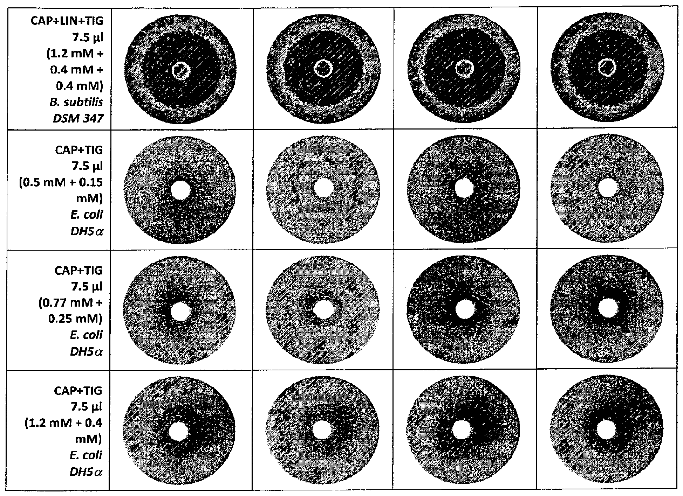

### Figure 3a: Antibiotic effect is not altered by combination of antibiotics and co-administration of pyruvate, uridine or pyruvate + urine.

The panel displays the non-growth zone of bacteria (*B. subtilis* DSM 347 or *E*. *coli* DH5α) that is caused by antibiotic diffusion from the soaked paper-pads into the growth plate. The size of the non-growth zone is a measure for antibiotic activity. The following antibiotic combinations were used: CAP + LIN, CAP + LIN + TIG, CAP + TIG.

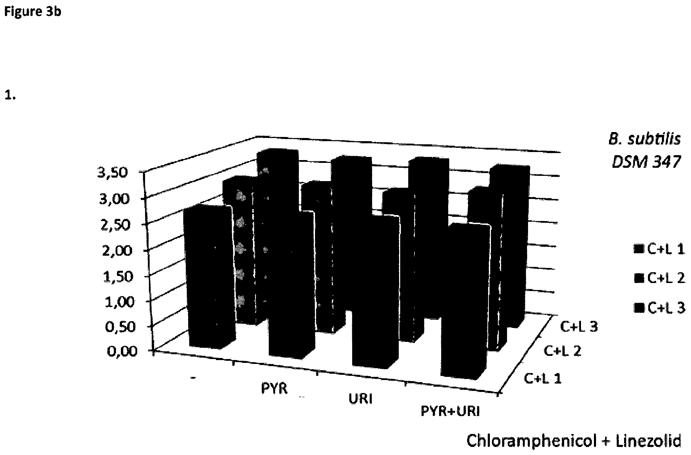

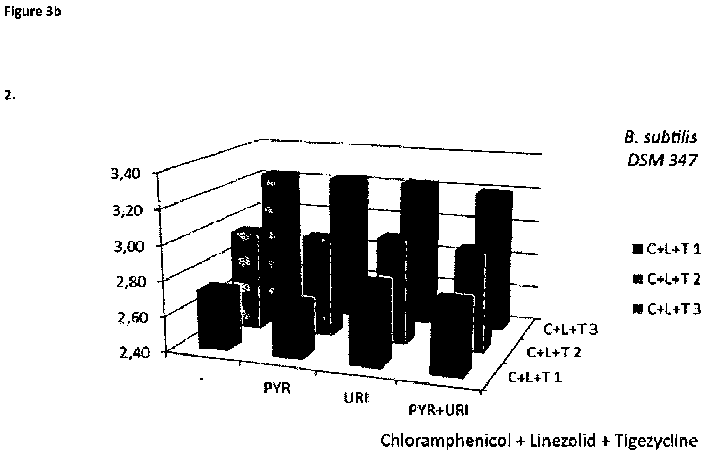

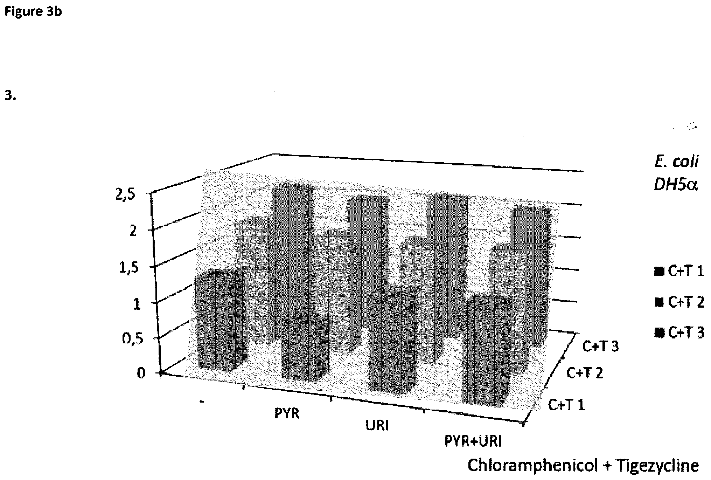

### Figure 3b: Quantitative analysis of the antibiotic combination effect (CAP+LIN; CAP+LIN+TIG; CAP+TIG) during co-administration of pyruvate, uridine or pyruvate + uridine.

**First panel:** Antibiotic effect of chloramphenicol + linezolid (C + L 1: 0.5 mM CAP + 0.15 mM LIN, C + L 2: 0.77 mM CAP + 0.25 mM LIN, C + L 3: 1.2 mM CAP + 0.4 mM LIN) on *B. subtilis* DSM 347. Antibiotic effect increases with concentration of antibiotic, but is independent of pyruvate, uridine or pyruvate + uridine supplementation.
**Second panel:** Antibiotic effect of chloramphenicol + linezolid + tigezycline (C + L +T 1: 0.5 mM CAP + 0.15 mM LIN + 0.15 mM TIG, C + L + T 2: 0.77 mM CAP + 0.25 mM LIN + 0.25 mM TIG, C + L + T 3: 1.2 mM CAP + 0.4 mM LIN + 0.4 mM TIG) on *B. subtilis* DSM 347. Antibiotic effect increases with concentration of antibiotic, but is independent of pyruvate, uridine or pyruvate + uridine supplementation.
**Third panel:** Antibiotic effect of chloramphenicol + tigezycline (C + T 1: 0.5 mM CAP + 0.15 mM TIG, C + T 2: 0.77 mM CAP + 0.25 mM TIG, C + T 3: 1.2 mM CAP + 0.4 mM TIG) on *E. coli* DH5α. Antibiotic effect increases with concentration of antibiotic, but is independent of pyruvate, uridine or pyruvate + uridine supplementation.

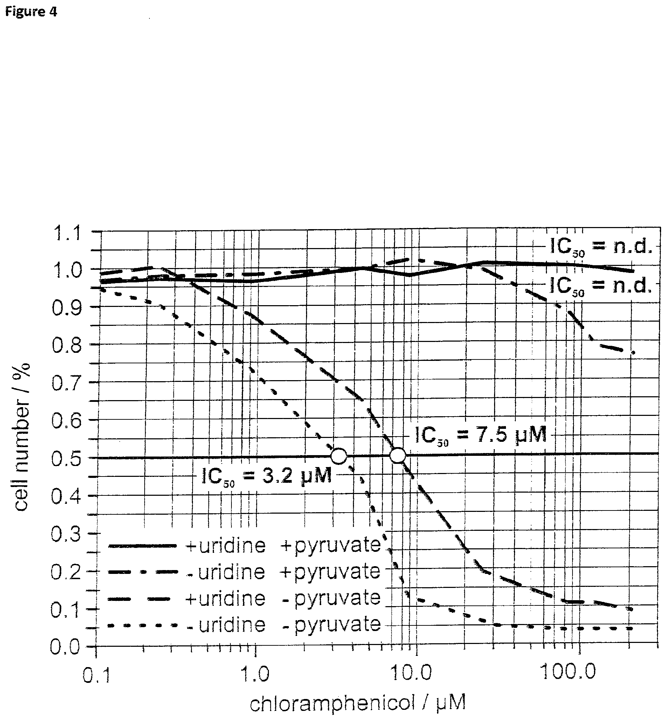

### Figure 4: Determination of IC₅₀ values of chloramphenicol in the presence or absence of pyruvate and/or uridine.

Vitality of 143B.TK⁻ cells was tested using resazurin (see example 4). The image displays a plot of cell number/% vs the chloramphenicol concentration /µM. It can be easily seen, that cells without uridine and pyruvate supplementation experienced a severe toxicity. IC₅₀ could be determined at a concentration of approximately 3.2 µM chloramphenicol. Cultures complemented with uridine experienced a shift of IC₅₀ towards approximately 7.5 µM chloramphenicol, indicating a stabilization of cell vitality. When complementation was carried out with pyruvate or pyruvate and uridine, both IC₅₀ values could not be determined, because they were beyond scale (IC₅₀ >300 µM chloramphenicol). Thus, in the plotted concentration range, uridine and pyruvate supplementation neutralized completely the side effects that are caused by chloramphenicol treatment without additives. Cell's vitality was indistinguishable from cell growth without chloramphenicol treatment.

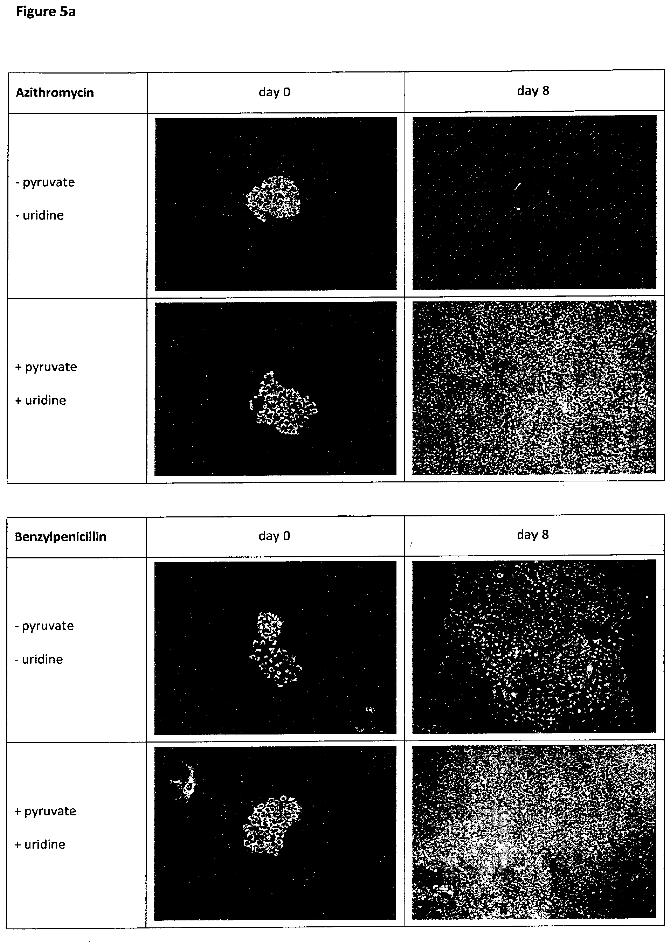

### Figure 5a: Side effect of azithromycin and benzylpenicillin on dividing human cells

***Top panel:*** The panel displays growth of a human cell line by phase contrast microscopy in presence of *azithromycin.* Cells were seeded on petri-dishes (day 0) and cell growth was monitored on a daily basis until day 8 of the timeline.
***Upper row:*** Without supplementation of pyruvate and uridine, the side effect of *azithromycin* is displayed, as cell growths retards and ultimately comes to a halt (day 8).
***Lower row:*** With supplementation of pyruvate and uridine, the side effect of *azithromycin* is completely abolished, as cell growths is indistinguishable from untreated cells (day 8).
***Lower panel:*** The panel displays growth of a human cell line by phase contrast microscopy in presence of *benzylpenicillin.* Cells were seeded on petri-dishes (day 0) and cell growth was monitored on a daily basis until day 8 of the timeline.
***Upper row:*** Without supplementation of pyruvate and uridine, the side effect of *benzylpenicillin* is displayed, as cell growths retards and ultimately comes to a halt (day 8).
***Lower row:*** With supplementation of pyruvate and uridine, the side effect of *benzylpenicillin* is completely abolished, as cell growths is indistinguishable from untreated cells (day 8).

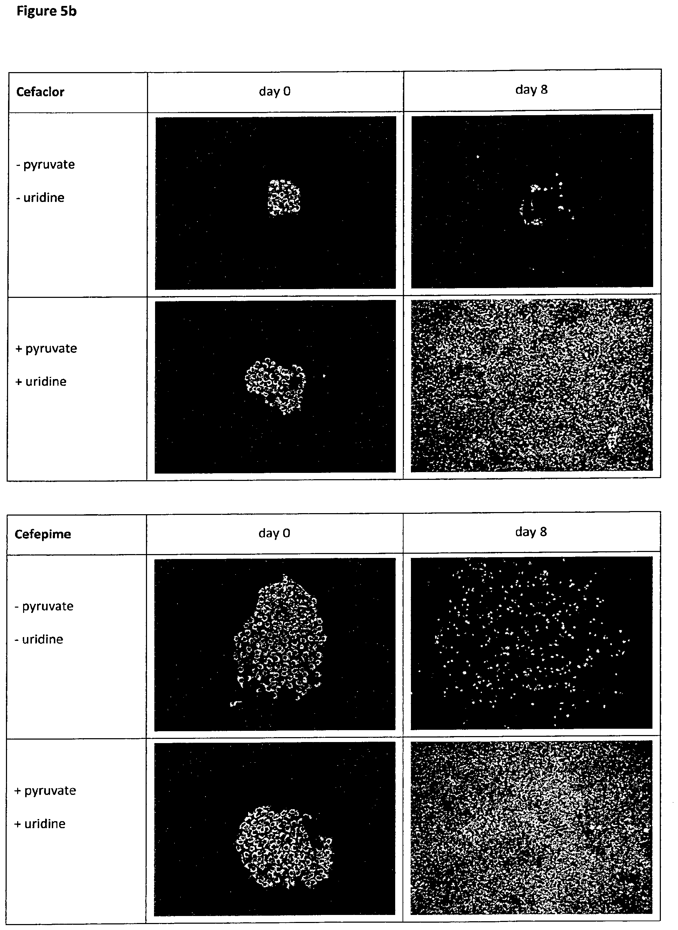

### Figure 5b: Side effect of cefaclor and cefepime on dividing human cells

***Top panel:*** The panel displays growth of a human cell line by phase contrast microscopy in presence of *cefaclor.* Cells were seeded on petri-dishes (day 0) and cell growth was monitored on a daily basis until day 8 of the timeline.
***Upper row:*** Without supplementation of pyruvate and uridine, the side effect of *cefaclor* is displayed, as cell growths retards and ultimately comes to a halt (day 8).
***Lower row:*** With supplementation of pyruvate and uridine, the side effect of *cefaclor* is completely abolished, as cell growths is indistinguishable from untreated cells (day 8).
***Lower panel:*** The panel displays growth of a human cell line by phase contrast microscopy in presence of *cefepime.* Cells were seeded on petri-dishes (day 0) and cell growth was monitored on a daily basis until day 8 of the timeline.
***Upper row:*** Without supplementation of pyruvate and uridine, the side effect of *cefepime* is displayed, as cell growths retards and ultimately comes to a halt (day 8).
***Lower row:*** With supplementation of pyruvate and uridine, the side effect of *cefepime* is completely abolished, as cell growths is indistinguishable from untreated cells (day 8).

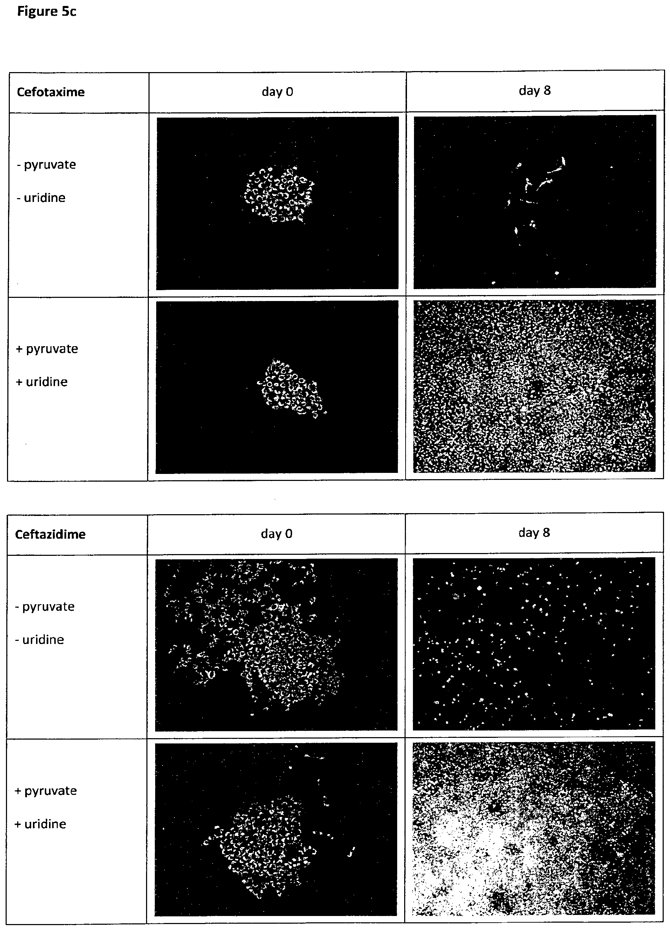

### Figure 5c: Side effect of cefotaxime and ceftazidime on dividing human cells

***Top panel:*** The panel displays growth of a human cell line by phase contrast microscopy in presence of *cefofaxime.* Cells were seeded on petri-dishes (day 0) and cell growth was monitored on a daily basis until day 8 of the timeline.
***Upper row:*** Without supplementation of pyruvate and uridine, the side effect of *cefotaxime* is displayed, as cell growths retards and ultimately comes to a halt (day 8).
***Lower row:*** With supplementation of pyruvate and uridine, the side effect of *cefotaxime* is completely abolished, as cell growths is indistinguishable from untreated cells (day 8).
***Lower panel:*** The panel displays growth of a human cell line by phase contrast microscopy in presence of *ceftazidime.* Cells were seeded on petri-dishes (day 0) and cell growth was monitored on a daily basis until day 8 of the timeline.
***Upper row:*** Without supplementation of pyruvate and uridine, the side effect of *ceftazidime* is displayed, as cell growths retards and ultimately comes to a halt (day 8).
***Lower row:*** With supplementation of pyruvate and uridine, the side effect of *ceftazidime* is completely abolished, as cell growths is indistinguishable from untreated cells (day 8).

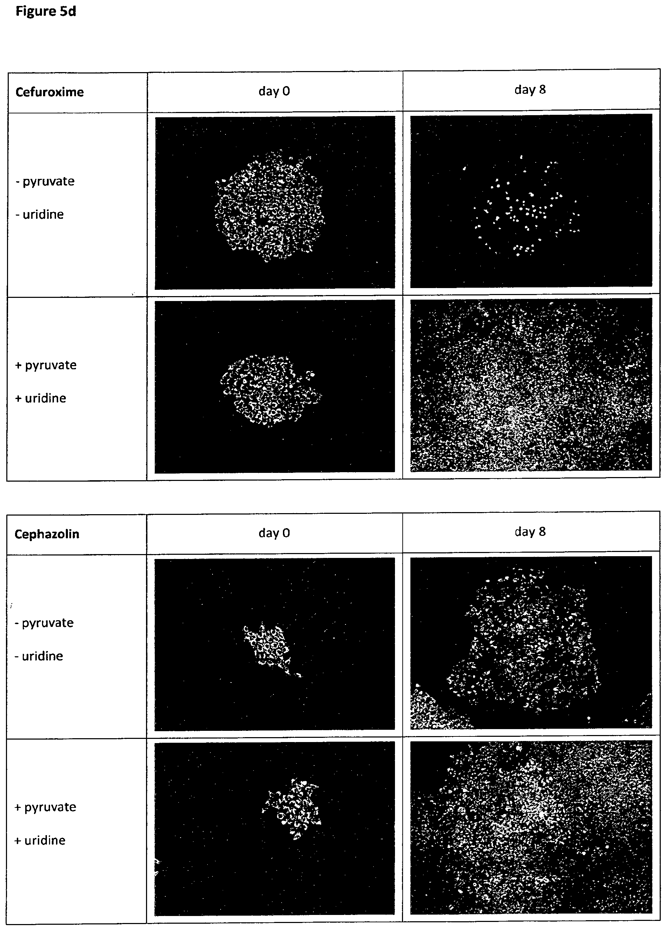

### Figure 5d: Side effect of cefuroxime and cephazolin on dividing human cells

***Top panel:*** The panel displays growth of a human cell line by phase contrast microscopy in presence of *cefuroxime.* Cells were seeded on petri-dishes (day 0) and cell growth was monitored on a daily basis until day 8 of the timeline.
***Upper row:*** Without supplementation of pyruvate and uridine, the side effect of *cefuroxime* is displayed, as cell growths retards and ultimately comes to a halt (day 8).
***Lower row:*** With supplementation of pyruvate and uridine, the side effect of *cefuroxime* is completely abolished, as cell growths is indistinguishable from untreated cells (day 8).
***Lower panel:*** The panel displays growth of a human cell line by phase contrast microscopy in presence of *cephazolin.* Cells were seeded on petri-dishes (day 0) and cell growth was monitored on a daily basis until day 8 of the timeline.
***Upper row:*** Without supplementation of pyruvate and uridine, the side effect of *cephazolin* is displayed, as cell growths retards and ultimately comes to a halt (day 8).
***Lower row:*** With supplementation of pyruvate and uridine, the side effect of *cephazolin* is completely abolished, as cell growths is indistinguishable from untreated cells (day 8).

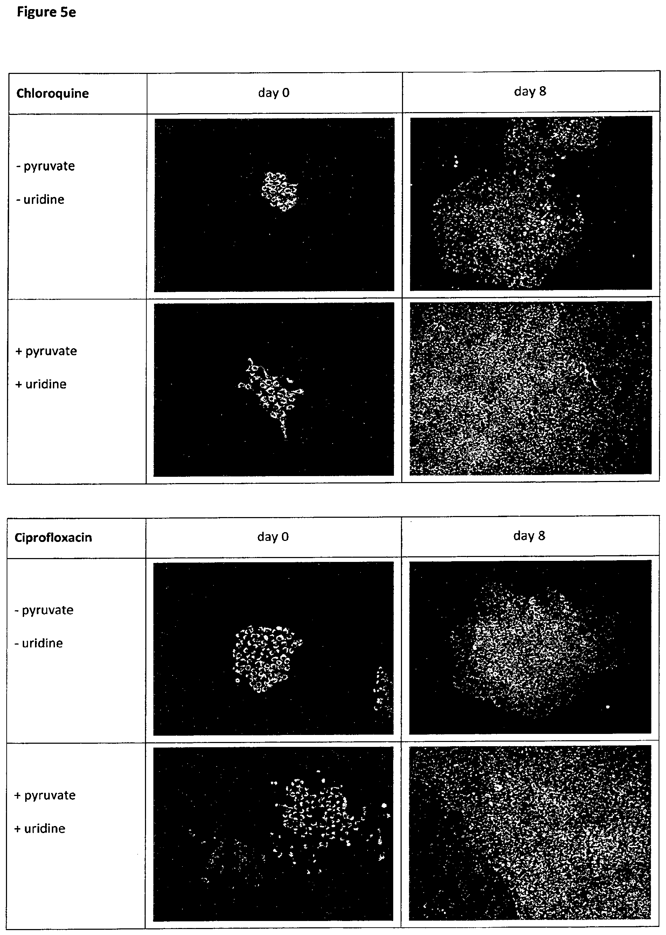

### Figure 5e: Side effect of chloroquine and ciprofloxacin on dividing human cells

***Top panel:*** The panel displays growth of a human cell line by phase contrast microscopy in presence of *chloroquine.* Cells were seeded on petri-dishes (day 0) and cell growth was monitored on a daily basis until day 8 of the timeline.
***Upper row:*** Without supplementation of pyruvate and uridine, the side effect of *chloroquine* is displayed, as cell growths retards and ultimately comes to a halt (day 8).
***Lower row:*** With supplementation of pyruvate and uridine, the side effect of *chloroquine* is completely abolished, as cell growths is indistinguishable from untreated cells (day 8).
***Lower panel:*** The panel displays growth of a human cell line by phase contrast microscopy in presence of *ciprofloxacin.* Cells were seeded on petri-dishes (day 0) and cell growth was monitored on a daily basis until day 8 of the timeline.
***Upper row:*** Without supplementation of pyruvate and uridine, the side effect of *ciprofloxacin* is displayed, as cell growths retards and ultimately comes to a halt (day 8).
***Lower row:*** With supplementation of pyruvate and uridine, the side effect of *ciprofloxacin* is completely abolished, as cell growths is indistinguishable from untreated cells (day 8).

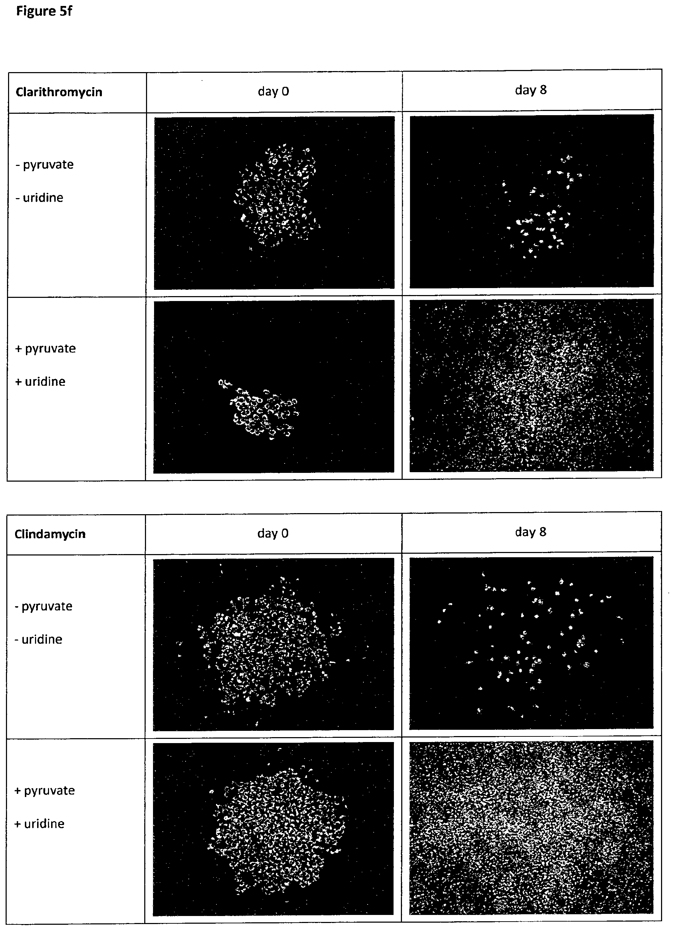

### Figure 5f: Side effect of clarithromycin and clindamycin on dividing human cells

***Top panel:*** The panel displays growth of a human cell line by phase contrast microscopy in presence of *clarithromycin.* Cells were seeded on petri-dishes (day 0) and cell growth was monitored on a daily basis until day 8 of the timeline.
***Upper row:*** Without supplementation of pyruvate and uridine, the side effect of *clarithromycin* is displayed, as cell growths retards and ultimately comes to a halt (day 8).
***Lower row:*** With supplementation of pyruvate and uridine, the side effect of *clarithromycin* is completely abolished, as cell growths is indistinguishable from untreated cells (day 8).
***Lower panel:*** The panel displays growth of a human cell line by phase contrast microscopy in presence of *clindamycin.* Cells were seeded on petri-dishes (day 0) and cell growth was monitored on a daily basis until day 8 of the timeline.
***Upper row:*** Without supplementation of pyruvate and uridine, the side effect of *clindamycin* is displayed, as cell growths retards and ultimately comes to a halt (day 8).
***Lower row:*** With supplementation of pyruvate and uridine, the side effect of *clindamycin* is completely abolished, as cell growths is indistinguishable from untreated cells (day 8).

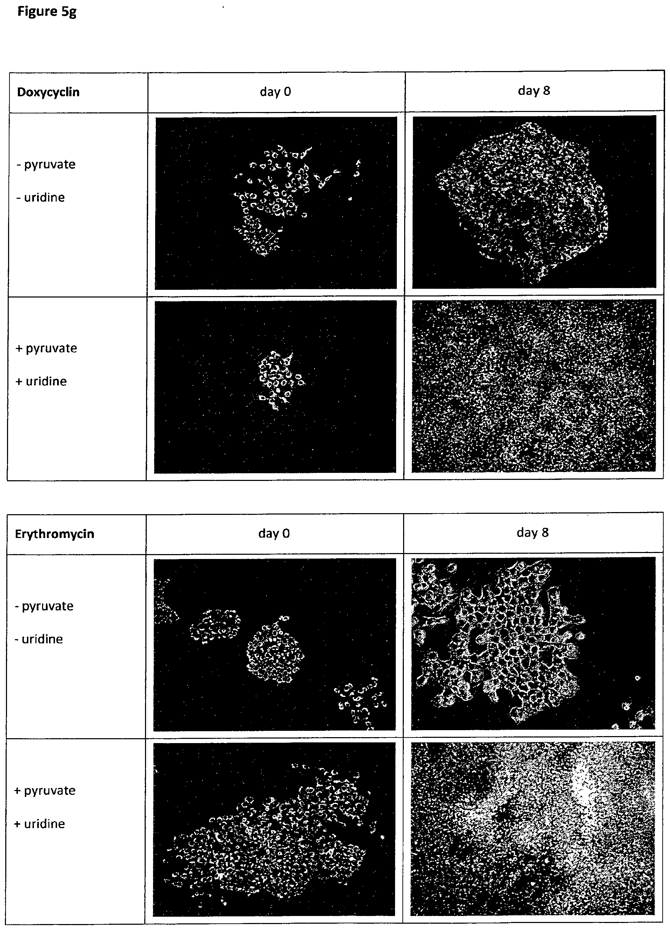

### Figure 5g: Side effect of doxycyclin and erythromycin on dividing human cells

***Top panel:*** The panel displays growth of a human cell line by phase contrast microscopy in presence of *doxycyclin.* Cells were seeded on petri-dishes (day 0) and cell growth was monitored on a daily basis until day 8 of the timeline.
***Upper row:*** Without supplementation of pyruvate and uridine, the side effect of *doxycyclin* is displayed, as cell growths retards and ultimately comes to a halt (day 8).
***Lower row:*** With supplementation of pyruvate and uridine, the side effect of *doxycyclin* is completely abolished, as cell growths is indistinguishable from untreated cells (day 8).
***Lower panel:*** The panel displays growth of a human cell line by phase contrast microscopy in presence of *erythromycin.* Cells were seeded on petri-dishes (day 0) and cell growth was monitored on a daily basis until day 8 of the timeline.
***Upper row:*** Without supplementation of pyruvate and uridine, the side effect of *erythromycin* is displayed, as cell growths retards and ultimately comes to a halt (day 8).
***Lower row:*** With supplementation of pyruvate and uridine, the side effect of *erythromycin* is completely abolished, as cell growths is indistinguishable from untreated cells (day 8).

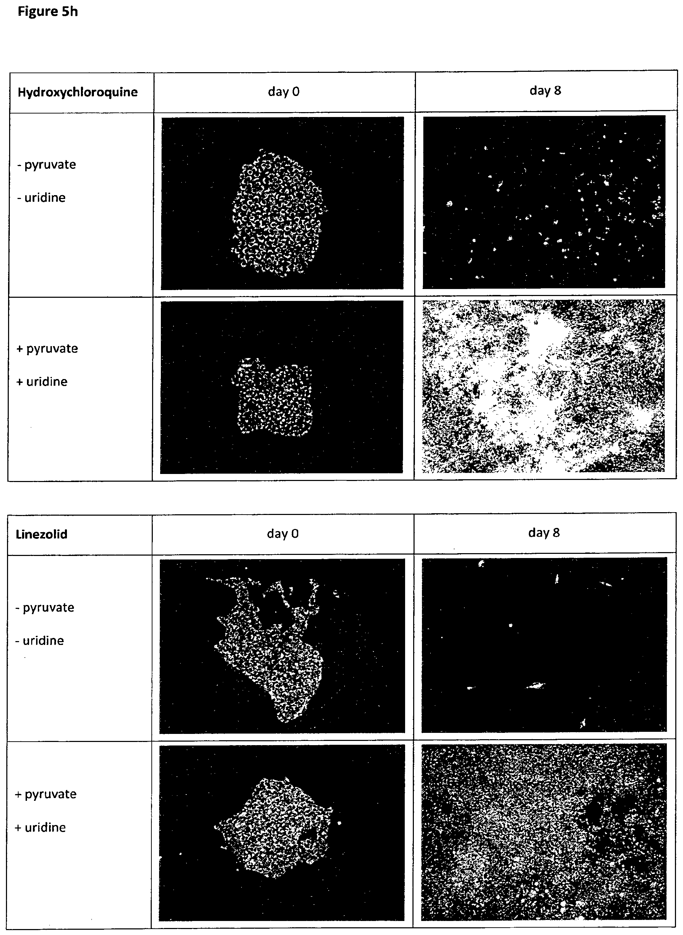

### Figure 5h: Side effect of hydroxychloroquine and linezolid on dividing human cells

***Top panel:*** The panel displays growth of a human cell line by phase contrast microscopy in presence of *hydroxychloroquine.* Cells were seeded on petri-dishes (day 0) and cell growth was monitored on a daily basis until day 8 of the timeline.
***Upper row:*** Without supplementation of pyruvate and uridine, the side effect of *hydroxychloroquine* is displayed, as cell growths retards and ultimately comes to a halt (day 8).
***Lower row:*** With supplementation of pyruvate and uridine, the side effect of *hydroxychloroquine* is completely abolished, as cell growths is indistinguishable from untreated cells (day 8).
***Lower panel:*** The panel displays growth of a human cell line by phase contrast microscopy in presence of *linezolid.* Cells were seeded on petri-dishes (day 0) and cell growth was monitored on a daily basis until day 8 of the timeline.
***Upper row:*** Without supplementation of pyruvate and uridine, the side effect of *linezolid* is displayed, as cell growths retards and ultimately comes to a halt (day 8).
***Lower row:*** With supplementation of pyruvate and uridine, the side effect of *linezolid* is completely abolished, as cell growths is indistinguishable from untreated cells (day 8).

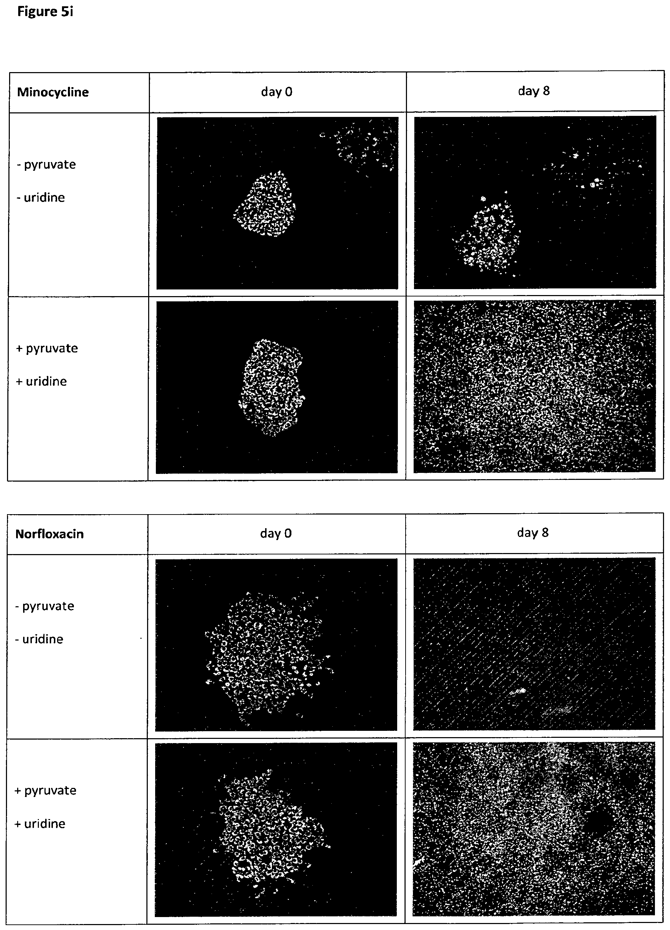

### Figure 5i: Side effect of minocycline and norfloxacin on dividing human cells

***Top panel:*** The panel displays growth of a human cell line by phase contrast microscopy in presence of *minocycline.* Cells were seeded on petri-dishes (day 0) and cell growth was monitored on a daily basis until day 8 of the timeline.
***Upper row:*** Without supplementation of pyruvate and uridine, the side effect of *minocycline* is displayed, as cell growths retards and ultimately comes to a halt (day 8).
***Lower row:*** With supplementation of pyruvate and uridine, the side effect of *minocycline* is completely abolished, as cell growths is indistinguishable from untreated cells (day 8).
***Lower panel:*** The panel displays growth of a human cell line by phase contrast microscopy in presence of *norfloxacin* Cells were seeded on petri-dishes (day 0) and cell growth was monitored on a daily basis until day 8 of the timeline.
***Upper row:*** Without supplementation of pyruvate and uridine, the side effect of *norfloxacin* is displayed, as cell growths retards and ultimately comes to a halt (day 8).
***Lower row:*** With supplementation of pyruvate and uridine, the side effect of *norfloxacin* is completely abolished, as cell growths is indistinguishable from untreated cells (day 8).

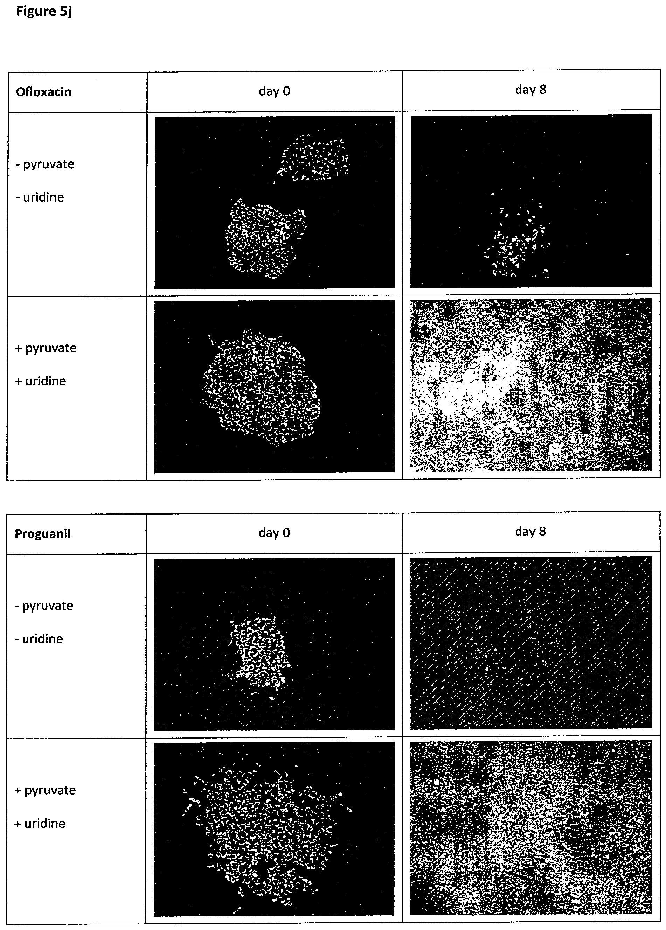

### Figure 5j: Side effect of ofloxacin and proguanil on dividing human cells

***Top panel:*** The panel displays growth of a human cell line by phase contrast microscopy in presence of *ofloxacin.* Cells were seeded on petri-dishes (day 0) and cell growth was monitored on a daily basis until day 8 of the timeline.
***Upper row:*** Without supplementation of pyruvate and uridine, the side effect of *ofloxacin* is displayed, as cell growths retards and ultimately comes to a halt (day 8).
***Lower row:*** With supplementation of pyruvate and uridine, the side effect of *ofloxacin* is completely abolished, as cell growths is indistinguishable from untreated cells (day 8).
***Lower panel:*** The panel displays growth of a human cell line by phase contrast microscopy in presence of *proguanil.* Cells were seeded on petri-dishes (day 0) and cell growth was monitored on a daily basis until day 8 of the timeline.
***Upper row:*** Without supplementation of pyruvate and uridine, the side effect of *proguanil* is displayed, as cell growths retards and ultimately comes to a halt (day 8).
***Lower row:*** With supplementation of pyruvate and uridine, the side effect of *proguanil* is completely abolished, as cell growths is indistinguishable from untreated cells (day 8).

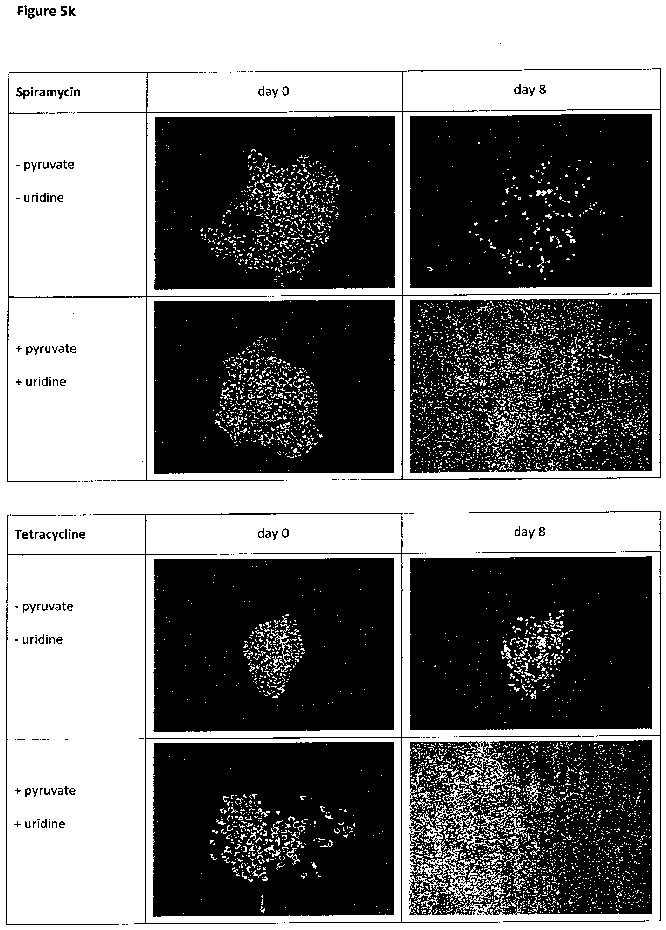

### Figure 5k: Side effect of spiramycin and tetracycline on dividing human cells

***Top panel:*** The panel displays growth of a human cell line by phase contrast microscopy in presence of *spiramycin.* Cells were seeded on petri-dishes (day 0) and cell growth was monitored on a daily basis until day 8 of the timeline.
***Upper row:*** Without supplementation of pyruvate and uridine, the side effect of *spiramycin* is displayed, as cell growths retards and ultimately comes to a halt (day 8).
***Lower row:*** With supplementation of pyruvate and uridine, the side effect of *spiramycin* is completely abolished, as cell growths is indistinguishable from untreated cells (day 8).
***Lower panel:*** The panel displays growth of a human cell line by phase contrast microscopy in presence of *tetracycline.* Cells were seeded on petri-dishes (day 0) and cell growth was monitored on a daily basis until day 8 of the timeline.
***Upper row:*** Without supplementation of pyruvate and uridine, the side effect of *tetracycline* is displayed, as cell growths retards and ultimately comes to a halt (day 8).
***Lower row:*** With supplementation of pyruvate and uridine, the side effect of *fefracycline* is completely abolished, as cell growths is indistinguishable from untreated cells (day 8).

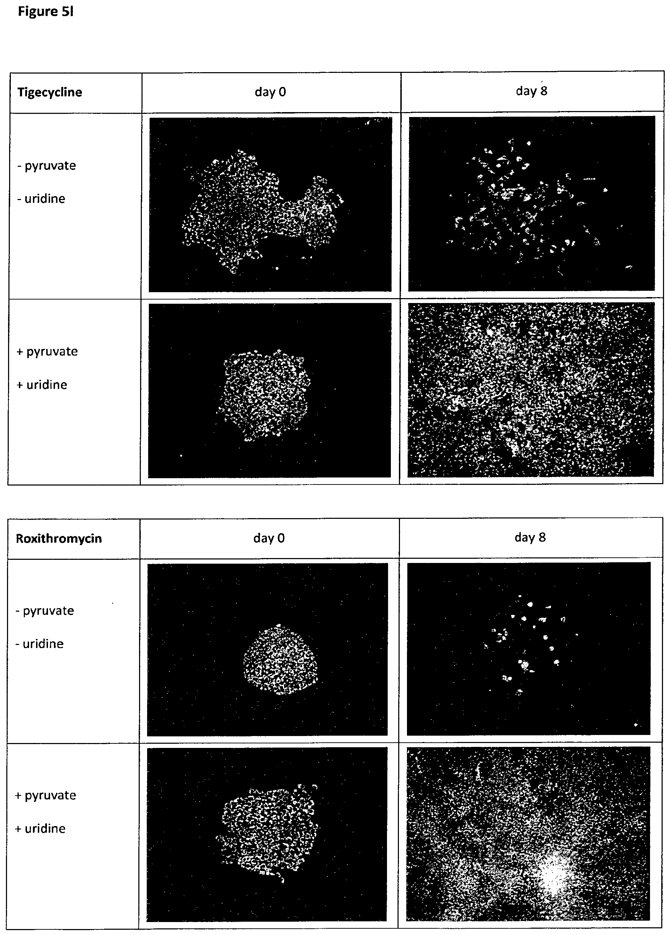

### Figure 5l: Side effect of tigecycline and roxithromycin on dividing human cells

***Top panel:*** The panel displays growth of a human cell line by phase contrast microscopy in presence of *tigecycline.* Cells were seeded on petri-dishes (day 0) and cell growth was monitored on a daily basis until day 8 of the timeline.
***Upper row:*** Without supplementation of pyruvate and uridine, the side effect of *tigecycline* is displayed, as cell growths retards and ultimately comes to a halt (day 8).
***Lower row:*** With supplementation of pyruvate and uridine, the side effect of *tigecycline* is completely abolished, as cell growths is indistinguishable from untreated cells (day 8).
***Lower panel:*** The panel displays growth of a human cell line by phase contrast microscopy in presence of *roxithromycin.* Cells were seeded on petri-dishes (day 0) and cell growth was monitored on a daily basis until day 8 of the timeline.
***Upper row:*** Without supplementation of pyruvate and uridine, the side effect of *roxithromycin,* is displayed, as cell growths retards and ultimately comes to a halt (day 8).
***Lower row:*** With supplementation of pyruvate and uridine, the side effect of *roxithromycin* is completely abolished, as cell growths is indistinguishable from untreated cells (day 8).

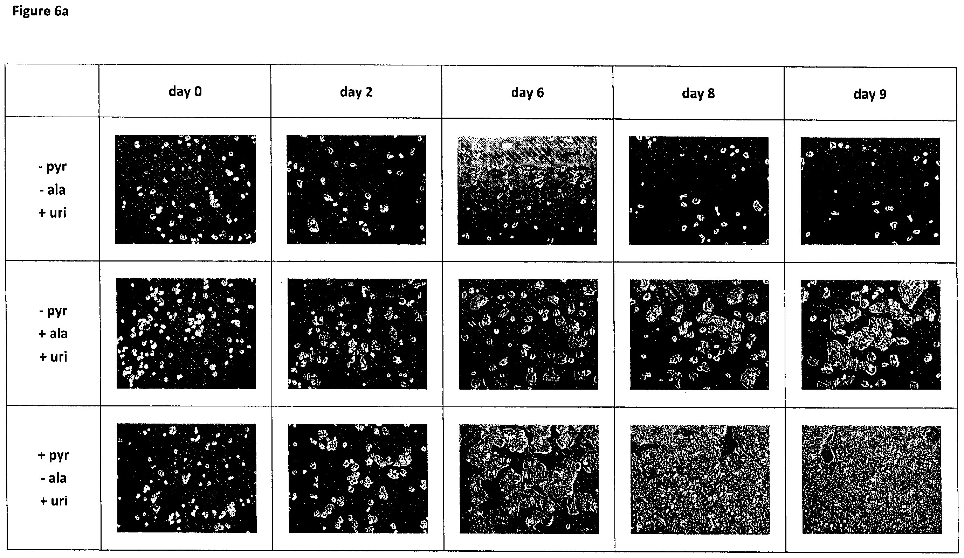

### Figure 6a: Alternative pyruvate source to ameliorate side effects in antibiotic therapy

The panel displays growth of a human HT29 rho zero cell line by phase contrast microscopy in presence of different supplementations. Cell growth was monitored on a daily basis until day 9 of the timeline.
***Upper row:*** In presence of uridine without any pyruvate source, cell growth stalls and comes to an halt (day 9).
***Middle row:*** When supplementation includes besides uridine a pyruvate source as for example alanine, cells prosper and grow almost in the same range as normal cells so that side effects of antibiotcs are minimized.
***Lower row:*** When supplementation includes pyruvate and uridine, the side effect of diminished cell growths is completely abolished and cells are indistinguishable from untreated normal cells.

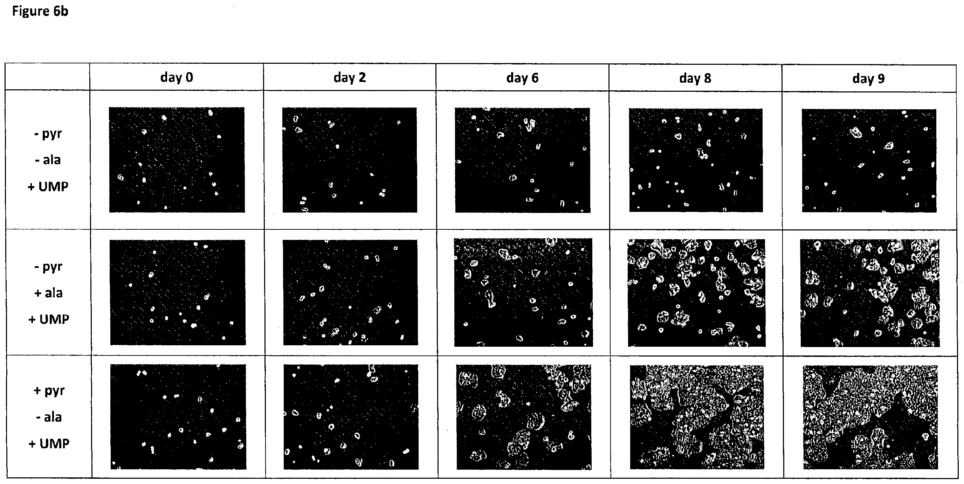

### Figure 6b: Alternative uridine source to ameliorate side effects in antibiotic therapy

The panel displays growth of a human HT29 rho zero cell line by phase contrast microscopy in presence of different supplementations. Cell growth was monitored on a daily basis until day 9 of the timeline.
***Upper row:*** In presence of UMP without any pyruvate source, cell growth stalls and comes to an halt (day 9).
***Middle row:*** When supplementation includes besides UMP a pyruvate source as for example alanine, cells prosper and grow almost in the same range as normal cells so that side effects of antibiotcs are minimized.
***Lower row:*** When supplementation includes UMP and pyruvate, the side effect of diminished cell growths is completely abolished and cells are indistinguishable from untreated normal cells.

### Abbreviations:

CAP: Chloramphenicol
LIN: Linezolid
TIG: Tigezycline

### Detailed Description of the Present Invention

The invention provides a composition comprising:
(iii) a therapeutically effective amount of at least one antibiotic; and
(iv) a therapeutically effective amount of both an uridine compound and pyruvate compound
   for use as a medicament, wherein
   the uridine compound is uridine, uracil, uridine phosphate(s) (e.g. uridine-5' monophosphate (UMP), uridine-5' diphosphate (UDP), uridine-5' triphosphate (UTP)) or salts thereof, UDP-glucose, cytidine-5' monophosphate (CMP), cytidine-5' diphosphate (CDP), CDP-glucose, orotic acid, orotidylate, acylated uridine or cytidine-diphosphocholine; and
   the pyruvate compound is pyruvic acid or a salt thereof (e.g. methyl pyruvate, ethyl pyruvate or sodium salt of pyruvate), creatine pyruvate, phosphoenolpyruvate, 2-phosphoglycerate, 3-phosphoglycerate, 1,3-biphosphoglycerate, glycerinaldehyde 3-phosphate, dihydroxyacetone phosphate, alanine, serine, cystein, glycine, tryptophan, asparagine, aspartate, methionine, malate or oxalacetate.
The invention further provides a pharmaceutical composition comprising
(iii) a therapeutically effective amount of at least one antibiotic; and
(iv) a therapeutically effective amount of both an uridine compound and a pyruvate compound
   for use in preventing or minimizing adverse side effects in the antibiotic therapy of bacterial infections in a subject, wherein
   the uridine compound is uridine, uracil, uridine phosphate(s) (e.g. uridine-5' monophosphate (UMP), uridine-5' diphosphate (UDP), uridine-5' triphosphate (UTP)) or salts thereof, UDP-glucose, cytidine-5' monophosphate (CMP), cytidine-5' diphosphate (CDP), CDP-glucose, orotic acid, orotidylate, acylated uridin or cytidine-diphosphocholine; and
   the pyruvate compound is pyruvic acid or a salt thereof (e.g. methyl pyruvate, ethyl pyruvate or sodium salt of pyruvate), creatine pyruvate, phosphoenolpyruvate, 2-phosphoglycerate, 3-phosphoglycerate, 1,3-biphosphoglycerate, glycerinaldehyde 3-phosphate, dihydroxyacetone phosphate, alanine, serine, cystein, glycine, tryptophan, asparagine, aspartate, methionine, malate or oxalacetate.

In the present invention the terms "formulation" and "(pharmaceutical) composition" are used interchangeably.

"Subject" as used herein refers to humans and non-human primates (e.g. guerilla, macaque, marmoset), livestock animals (e.g. sheep, cow, horse, donkey, pig), companion animals (e.g. dog, cat), laboratory test animals (e.g. mouse, rabbit, rat, guinea pig, hamster), and any other organism who can be benefit from the agents of the present disclosure. There is no limitation on the type of animal that could benefit from the presently described agents. A subject regardless of whether it is a human or non-human organism may be referred to also as a patient, individual, animal, host, or recipient.

The term "about" as used herein, means in quantitative terms plus or minus 5%, or in another embodiment plus or minus 10%, or in another embodiment plus or minus 15%, or in another embodiment plus or minus 20%.

It has been found out by the inventors that it is possible to ameliorate and/or eliminate the reported side effects of antibiotics if the administration of antibiotics is accompanied by the supplementation with an uridine compound and pyruvate compound as defined above. The term "an uridine and pyruvate compound" means a mixture of both of them. It also encompasses two or more of each of them or mixtures or two or more of each.

The most common side effects of antibiotic therapy to be diminished and/or eliminated are: rash, diarrhea, abdominal pain, nausea/vomiting, drug fever, hypersensitivity (allergic) reactions, serum sickness, vaginal candidiasis, renal (kidney) toxicity, ototoxicity (hearing loss), dizziness, nystagmus, headache, liver toxicity, eosinophilia (elevated white blood cells), anorexia, hemolytic anemia, peripheral neuropathy, dizziness, reddish-orange body fluids, flushing, hypotension, itching, phlebitis, taste, taste alterations, photosensitivity , tooth discoloration in children, lethargy, insomnia, photosensitivity , pseudomembranous colitis, jaundice, metallic taste.

Unexpectedly, it has been found that the combination of uridine and pyruvate compounds and one or more antibiotics results in no significant diminishment of the effectiveness of the antibiotic(s), or alteration of the pharmacokinetics of the antibiotic.

According to the present invention any antibiotic may be used. Preferentially, those antibiotics can be selected that belong to the group of protein synthesis inhibitors or those presenting with side-effects related to the activity of cellular protein synthesis. Suitable antibiotics include those of the classes aminoglycosides, ansamycins, carbacephmes, cephalosporins, glycopeptides, macrolides, penicillins, polypeptides, quinolones, sulfonamides, tetracyclines, glycycyclines, oxazolidinones, amphenicols, pleuromutilins, lincosamides, streptogramins, steroid antibacterials, cyclopeptides lipopeptides, and mixtures thereof.

Specific antibiotics that may be used (alone or in combination) include, for example, amikacin, gentamicin, kanamycin, neomycin, netilmicin, streptomycin, tobramycin, paromomycin, geldanamycin, herbimycin, loracarbef, ertapenem, doripenem, imipenem/cilastatin, meropenem, cefadroxil, duricef, cefazolin, cefalotin, cefalothin, cefalexin, cefaclor, cefamandole, cefoxitin, cefprozil, cefuroxime, cefixime, cefdinir, cefditoren, cefoperazone, cefotaxime, cefpodoxime, ceftazidime, ceftibuten, ceftizoxime, ceftriaxone, cefepime, ceftobiprole, teicoplanin, vancomycin, azithromycin, clarithromycin, dirithromycin, erythromycin, roxithromycin, troleandomycin, telithromycin, spectinomycin, aztreonam, amoxicillin, ampicillin, azlocillin, carbenicillin, cloxacillin, dicloxacillin, flucloxacillin, mezlocillinm, meticillin, nafcillin, oxacillin, penicillin, piperacillin, ticarcillin, ciprofloxacin, enoxacin, gatifloxacin, levofloxacin, lomefloxacin, fodaximicin, moxifloxacin, norfloxacin, ofloxacin, trovafloxacin, grepafloxacin, sparfloxacin, temafloxacin, mafenide, sulfonamidochrysoidine sulfacetamide, sulfadiazine, ulfamethizole, sulfanilamide, sulfasalizine, sulfisoxazole, trimethoprim, trimethroprim-sulfamethoxazole (co-trimoxazole) (TMP-SMX), doxycycline, minocycline, oxytetracycline, tetracycline, chloramphenicol, tigezycline, linezolid, clindamycin, lincomycin, ethambutol, myambutol, fosfomycin, fusidic acid, furazolidone, isoniazid, linezolid, metronidazole, mupirocin, nitrofurantoin, platensimycin, pyrazinamide, quinupristin/dalfopristin, rifampicin, thiamphenicol, tinidazole, dapsone, clofazimine dihydrostreptomycin, framycetin, ribostamycin, arbekacin, bekanamycin, dibekacin tobramycin, hygromycin B, sisomicin, isepamicin, verdamicin, astromicin, chlortetracycline clomocycline, demeclocycline, lymecycline, meclocycline, metacycline, penimepicycline, rolitetracycline, torezolid, eperezolid, posizolid, radezolid, azidamfenicol, florfenicol, retapamulin, tiamulin, valnemulin, flurithromycin, josamycin, midecamycin, miocamycin, oleandomycin, rokitamycin, spiramycin, tylosin, ketolides, cethromycin, solthromycin, pirlimycin, pristinamycin, virginiamycin, chloroquin, hydroxochloroquin, cefotaxim, polymyxin B, colistin, bacitracin, tyrotricin, gramicidin, daptomycin, fidaxomicin, and mixtures thereof.

In an embodiment, the formulation may include a combination of 2, of 3, of 4, of 5 or of 6 or more antibiotics or agents. The combination or single antibiotic(s) chosen will necessarily depend on the nature and type of infection that is to be treated in each circumstance and the medical particulars of the individual patient. Such selection is well within the scope of a person of skill in the art. In preferred embodiments of the present invention at least one of the antibiotics is selected from the subgroup of protein synthesis inhibitors or these antibiotics that display side-effects involved in protein synthesis.

In another embodiment of the present invention at least one of the antibiotics is selected from the subgroup of chloramphenicol, linezolid or tigezycline.

Antibiotic-treatable infections may include, for example, bacterial sinusitis, various sexually transmitted diseases of bacterial origin, pharyngitis, otitis, especially otitis media, bacterial bronchitis, bacterial pneumonia, diverticulitis, urinary tract invections, skin and skin structure infections, cellulitis, abscesses, furuncles, impetigo, pyoderma, wound infections, acne, nail infections, chronic bacterial prostatitis, acute pyelonephritis, and/or infections caused by any one or more of *Haemophilus influenza, Haemophilus parainfluenzae, Moraxella catarrhalis, Staphylococcus aureus, Staphylococcus epidermidis, Staphylococcos saprophyticus, Pseudomonas aeruginosa, Serratia marcescens, Escherichia coli, Klebsiella pneumonia, Streptococcus pneumonia, Streptococcus neumoniaeparainfluenzae, Streptococcus pyogenes, Chlamydia pneumonia, Legionella pneumophila, Mycoplasma pneumonia, Vibrio cholera, Bacillus anthracis, Eikenella corrodens, Neisseria gonorrhoeae, Enterococcus faecalis, Enterobacter cloacae, Proteus mirabilis,* bacteria of the genus *Bacteroides,* including *Bacteroides fragilis,* bacteria of the genus *Fusobacterium,* and bacteria of the genus *Peptostreptococcus,* and MRSA infections.

The dosage of the composition according to the invention, in particular of the antibiotic component, is determined by the physician on the basis of the patient-specific parameters, such as age, weight, sex, severity of the disease, *etc.* The mode of treatment and the dosage regimen of each (i) the antibiotic(s) and (ii) the uridine and pyruvate compounds will be mainly dictated by the specific antibiotic(s). In general, the unit dose and the dosage regimen will parallel that of the antibiotic if it were to be administered alone. For example, if the antibiotic recommended dosage is 200 mgs at two hour intervals, the antibiotic dosage in the formulation of the present invention will remain substantially the same, as would the frequency of administration of the entire formulation. However, the therapeutically effective dosage of any specific antibiotic or agent will vary somewhat from patient to patient, and will depend upon the condition of the patient and the dosage form.

The terms "dosage regimen" or "mode of treatment" refer to a timely sequential or simultaneous administration of the components (i) and (ii) of the composition of the present invention. This means that components (i) and (ii) may be provided in a unit dosage form with physical contact to each other (e.g. one single tablet or solution) or as separate entities (e.g. two tablets or solutions) to be taken simultaneously or with a certain time difference. This time difference may be between 0.5 hour and 1 day, preferably between 1 hour and 5 hours. In one embodiment, the (i) antibiotic(s) and (ii) uridine and/or pyruvate are physically segregated within the dosage form, for example, separated by an acrylic or cellulose membrane in a tablet or capsule. In an alternative embodiment, both ingredients (i) and (ii) are in physical contact within the dosage form, such as for example, in a mixed powder or granules form (free or formed into a tablet or capsule) or a liquid. The mode of administration of (i) and (ii) is preferably simultaneously, i.e. at substantially the same time, from separate entities. In a preferred embodiment, the antibiotic is administered via another administration way than the uridine and pyruvate compounds. In this regard it may be advantageous to administer either (i) the antibiotic or (ii) the uridine and pyruvate compounds intravenously and the other orally. If uridine and pyruvate compounds are administered as component (ii) they may be taken together or with a certain time difference. They may be provided in a single unit dosage form (e.g. one single tablet or solution) or as separate entities (e.g. two tablets or solutions) to be taken simultaneously or with a certain time difference. This time difference may be same as mentioned before.

In the present invention the uridine compound is administered to increase a level of uridine in a tissue, plasma, or cell of a subject.

In the present invention the term "uridine" or "uridine compound" means uridine, uridine phosphate(s) or uracil. Examples are poly-U, any C/U-containing oligonucleotide and RNA/DNA, respectively. In other words, the term "uridine (compound)" as used herein refers to any uridine phosphate or salt thereof. In one embodiment, the uridine compound that is administered in the present invention is a uridine-5'monophosphate (UMP). In another embodiment, the uridine is a uridine-5'-diphosphate (UDP). In another embodiment, the uridine is a uridine-5'triphosphate (UTP). In another embodiment, the uridine is UDP-glucose. Each possibility represents a separate embodiment of the present invention.

In another embodiment, the uridine compound is a cytidine-5'monophosphate. In another embodiment, the uridine compound that is administered is a cytidine-5'-diphosphate (CDP). In another embodiment, the uridine compound that is administered is a CDP-glucose. In another embodiment the uridine compound is e.g. orotic acid or orotidylate.

In other embodiments the uridine compound is acylated uridine.. In another embodiment, therapeutically or pharmacologically effective doses of acyl derivatives of uridine or mixtures thereof, e.g. those disclosed in U.S. Pat. No. 5,470,838, are administered.

In another embodiment, the uridine compound is cytidine-diphosphocholine (CDP-choline; citicholine).

In another embodiment, a salt of the uridine phosphate is utilized in the present invention, e.g. UMP disodium.

In another embodiment, a mixture of two or more of the above mentioned uridine compounds is administered.

In one embodiment, the uridine compound is administered in a dosage of between about 20 milligrams (mg) and 50 grams (g) per day. In another embodiment, the uridine compound is administered in a dosage of about 50 mg-30 g per day. In another embodiment, the dosage is about 75 mg-20 g; 100 mg-15 g; 100 mg-10 g; 200 mg-8 g; 400 mg-6 g; 600 mg-4 g; 800 mg-3 g; 1-2.5 g; 1.5-2 g, about 2 g per day.

According to the present invention the pyruvate compound is pyruvic acid, a salt thereof (e.g. methyl or ethyl pyruvate) or creatine pyruvate. Additionally, all compounds that can be easily converted within the cell to pyruvate, e. g. phosphoenolpyruvate, 2-phosphoglycerate, 3-phosphoglycerate, 1,3-biphosphoglycerate, glyceraldehyde 3-phosphate, dihydroxyacetone phosphate, alanine, serine, cystein, glycine, tryptophan, asparagine, aspartate, methionine, malate or oxalacetate can be utilized. In a preferred embodiment the pyruvate compound is sodium salt of pyruvate.

In one embodiment, the pyruvate compound is administered in a dosage of between about 100 milligrams (mg) and 10 grams (g) per day. In another embodiment, the pyruvate compound is administered in a dosage of about 200 mg-9 g per day. In another embodiment, the dosage is about 500 mg-8 g; 750 mg-7 g; 1-6.5g, 1.5-6 g, 2-5 g, or 2.5-3.5 g per day.

The pharmaceutical composition of the present invention may be prepared and administered in any dosage form suitable for administration to the subject's body. It is formulated to be compatible with its intended route of administration. Examples of routes of administration include parenteral (e.g. intravenous, intradermal, subcutaneous), oral, by inhalation, systemic (e.g. topical, transmucosal), and rectal administration.

In a preferred embodiment, the formulation may be prepared as a tablet, liquid, gel, a suspension, an emulsion or a solution.

Solutions or suspensions used for parenteral, intradermal, or subcutaneous application can include the following components: a sterile diluents such as water for injection, saline solution, fixed oils, polyethylene glycols, glycerine, propylene glycol, or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates, or phosphates and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes, or multiple dose vials made of glass or plastic.

Pharmaceutical compositions suitable for an injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor® EL (BASF, Parsippany, N.J.), or phosphase buffered saline (PBS). In all cases, the composition must be sterile and should be fluid to the extent that easy syringability exists. It should be stable under the conditions of manufacture and storage and must be preserved against the contaminating action of microorganism such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganism can be achieved by various antibacterial and antifungal agents, for example, parabens, chlorobutanol, phenol, ascorbic acid, thimerosal, and the like. According to embodiments, isotonic agents, for example, sugars, polyalcohols such as manitol, sorbitol, or sodium chloride in the composition are added. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preparation is prepared by vacuum drying or freeze-drying, which yields a powder of the active ingredient plus any additional desired ingredient from a previously sterile-filtered solution thereof.

In a further preferred embodiment the formulation of the present invention is suitable for oral administration. Oral compositions generally include an inert diluents or an edible carrier. For the purpose of oral therapeutic administration, the active compound can be incorporated with excipients and used in the form of tablets, troches, chewie, gel caps, soft gel or capsules, e.g. gelatin capsules. Oral compositions can also be prepared using a fluid carrier for use as a mouthwash. Pharmaceutically compatible binding agents, or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid. Primogel, or corn starch; a lubricant such as magnesium searate or sterotes; a glidant such as colloidal silicon dioxide; a sweetening agent such as sucrose or saccharin; or a flavoring agent such as peppermint, methyl salicylate, or strawberry, cherry, grape, lemon, or orange flavoring.

For administration by inhalation, the compounds are delivered in the form of an aerosol spray from pressured container or dispenser which contains a suitable propellant, e.g., a gas such as carbon dioxide, or a nebulizer.

According to embodiments, intravitreal injection is accomplished using PLGA-based microparticles or nanoparticules (liposomes). PEG-based formulas may also be used. Accordingly, the other methods for injectable pharmaceutical compositions are expressly contemplated for intravitreal injection.

Systemic administration can also be transmucosal or transdermal. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art. The compounds can also be prepared in the form of suppositories (e.g., with conventional suppository bases such as cocoa butter and other glycerides) or retention enemas for rectal delivery.

In addition to the other forms of delivery, the compounds are deliverable via eye drop or intraocular injection. With respect to eye drops, the compositions of the present disclosure optionally comprise one or more excipients intended for topical application to the eye or nose. Excipients commonly used in pharmaceutical compositions intended for topical application to the eyes, such as solutions or sprays, include, but are not limited to, tonicity agents, preservatives, chelating agents, buffering agents, surfactants and antioxidants. Suitable tonicity-adjusting agents include mannitol, sodium chloride, glycerin, sorbitol and the like. Suitable preservatives include p-hydroxybenzoic acid ester, benzalkonium chloride, benzododecinium bromide, polyquaternium-1 and the like. Suitable chelating agents include sodium edentate and the like. Suitable buffering agents include phosphates, borates, citrates, acetates and the like. Suitable surfactants include ionic and nonionic surfactants, though nonionic surfactants are preferred, such as polysorbates, polyethoxylated castor oil derivatives and oxyethylated tertiary octylphenol formaldehyde polymer (tyloxapol). Suitable antioxidants include sulfites, ascorbates, BHA and BHT.

The formulation of the present invention may include any additional component as desired, as long as such components do not substantially erode the effectiveness of the antibiotic or the uridine and pyruvate compounds. Such components may include, for example, thickeners, sweeteners, flavorants, fragrances, additional pharmaceuticals, glycine, mannitol, silicone dioxide, silica gels, binders, vitamins, carriers (such as, for example, glycerin, starches, celluloses, chitins, starches, water, alcohol) and minerals.

In the following particular preferred embodiment of the present invention are described. The life threating situation of a mitotic cell treated with an antibiotic can be rescued, when pyruvate and uridine compounds are administered simultaneously with the antibiotic. As described in detail in example 1 (Figure 1a-d), the adverse side effect of antibiotics can be ameliorated and/or prevented, when the antibiotic is co-administered with pyruvate and uridine. Example 1 shows also that in co-cultures of mitotic and resting cells (Figure 1b, 1c) the resting cells are not challenged by the antibiotic, because they do contain still enough OXPHOS complexes so that biochemical pathways are not altered. That the mitochondrial protein synthesis is the central point of antibiotic side effects is demonstrated by Figure 1d: Cells harboring an artificial mitochondrial CAP resistance mutation (red cells) are not influenced by CAP as they grow also in uridine and pyruvate deficient media. In contrary, the green cells (normal, cap sensitive cells) deteriorate when treated with CAP without uridine and pyruvate supplementation.

That the antibiotic effect is not affected by the co-administration of pyruvate and/or uridine is demonstrated by example 2: The antibiotic effect was tested in dependence of pyruvate, uridine or pyruvate + uridine complementation and was solely depending on the applied antibiotic dosage. Moreover, also a combination of antibiotics as exemplified in example 3 did not show any influence of the antibiotic capacity when supplementation with pyruvate, uridine or pyruvate + uridine occurred.

To prove whether or not the antibiotic effect is attenuated or diminished when pyruvate, uridine or pyruvate + uridine are co-administered, *B. subtilis* and *E*. *coli* strains were tested in the presence and absence of the supplementing compounds. It turned out (see example 2, Figure 2a and 2b) that the antibiotic effect is independent of co-administration of pyruvate, uridine or pyruvate + uridine. Moreover, even combinations of antibiotics in the presence of pyruvate, uridine or pyruvate + uridine did not alter the antibiotic effect of the drugs (see example 3, Figure 3a and 3b).

To determine the actual impact of antibiotic + supplementation by pyruvate, uridine or pyruvate + uridine onto cells performance, vitality tests were carried out utilizing resazurin (see example 4, Figure 4). This test resulted in a shift of IC₅₀ side-effect-values towards a far better tolerance of the drug. The IC₅₀ side-effect-values of chloramphenicol for example shifted from 3.2 µM of cells without supplementation to 7.5 µM of cells supplemented with uridine. Complementation with pyruvate or pyruvate + uridine yielded shifts that were beyond our scale (> 300 µM) and were practically indistinguishable from cell viability when cells were not treated with an antibiotic. Thus, administering antibiotics together with pyruvate, uridine or pyruvate + uridine leads to a complete loss of ameliorating or adverse side effects.

According to the present invention a formulation is provided where the antibiotic is administered with both an uridine and pyruvate compound since it has been found out that this combination is superior over using uridine or pyruvate alone. The reason is that each uridine and pyruvate alone reduce different aspects of the side-effects of antibiotics. The uridine compound is advantageous to provide an alternative source for uridine and dependent metabolic products as one side-effect of the antibiotics is the interruption of the uridine biosynthesis so that the cells suffer under slowed-down nucleic acid synthesis. The pyruvate compound is advantageous to refill the NAD+ level which allows the cell to run the glycolysis again, as this is the only remaining energy source if the respiratory chain is lost which is caused by another side-effect of the antibiotics. If both aspects of the side-effects are compensated the affected cells are able to maintain nucleic acid synthesis, especially for cell division and to keep the metabolism alive.

The following examples show preferred embodiments of the present invention but are not intended to limit the present invention in any aspect.

### Example 1:

### Side-effect of antibiotics and its neutralization

### A)

In order to understand the side effects of antibiotics, we incubated cultured human cells with antibiotics that are known to impede cell's metabolism. The experiments were carried out with chloramphenicol, tigezycline or linezolid under otherwise identical conditions. In details, 143B.TK⁻ cells were plated on petri-dishes and cultured in DMEM F0435 (w/o pyruvate), supplemented with 5% dialyzed fetal calf serum. Cells were treated with chloramphenicol (150 µg/ml culture medium, 450 µM; or linezolid, or tigezycline) and medium was exchanged on a daily basis. After 4 days of regular cell growth in the presence of the antibiotic, cell growth retarded and came to a halt after 6 days. After additional 4 days, cells ceased to exist (see figure 1a).

To understand the influence of the antibiotic on cell cycles state, a co-culture was established that was based on a primary fibroblasts monolayer (mimicking cells at resting state) that was inoculated with 143B.TK⁻ cells (mimicking cells in mitotic state, dividing cells). Under otherwise identical conditions, the co-cultures were subjected to the treatment with antibiotics (150 µg/ml culture medium, 450 µM; or linezolid, or tigezycline) in DMEM F0435 (w/o pyruvate), supplemented with 5% dialyzed fetal calf serum.

As seen in the above described experiment (Figure 1a), cell growth of the 143B.TK-cells came to a halt after 4-5 days and ceased to exist after additional 6 days (see figure 1b and 1c). However, the fibroblast layer was completely unaffected and vital to start regrowth again (proven by executing scratches through the monolayer; "wound-healing" set in immediately). When the same experiment was carried out in the presence of uridine and/or pyruvate under otherwise identical conditions, the cell growth of 143B.TK⁻ cells was completely unaffected, demonstrating that the observed side effect relies exclusively on energetic deficiency and/or cell cycle stalling. Both side-effects could be removed by co-administering uridine and/or pyruvate or derivatives thereof.

To see whether or not the side-effect of these antibiotics reside within the mitochondria of the cell, a co-culture was established that was made of a primary fibroblast monolayer inoculated with 143B.TK⁻ and 143.TK⁻CAP^{res} cells. The latter cell line is identical to the 143B.TK- cell line, except that these cells carry a CAP resistant mutation within the 16S rRNA gene located within the mitochondrial genome and are differently color coded. Upon chloramphenicol administration, the 143B.TK- cell line exhibited the same behavior as described above: cells stopped growth after 4-5 days and ceased to exist after additional 6 days whereas CAP-resistant cells exhibited no limitation in cell growth (see figure 1d). When these co-cultures were supplemented with pyruvate and/or uridine (pyruvate: 100 µg/ml sodium pyruvate; uridine: 50 µg/ml uridine; supplementation of growth medium) both 143B.TK⁻ cell lines were indistinguishable in their growth rate, demonstrating that the severe side-effect of the antibiotic was completely abolished.

### B)

As described in example 1 part A, similar experiments were carried out utilizing the antibiotics azithromycin, benzylpenicillin, cefaclor, cefeprime, cefotaxime, ceftazidime, cefuroxime, cephalozolin, chloroquine, ciprofloxacin, clarithromycin, clindamycin, doxycyclin, erythromycin, hydroxychloroquine, linezolid, minocycline, norfloxacin, ofloxacin, proguanil, spiramycin, tetracycline, tigecycline, roxithromycin in concentrations of 0.1 - 1000 µM under otherwise identical conditions. In details, 143B.TK⁻ cells, as well as IMR-32 (neuroblastoma), Calu-1 (lung epidermoid carcinoma), MCF-7 (invasive breast ductal carcinoma), A375 (malignant melanoma), HT1080 (fibrosarcoma), Panc-1 (pancreas epitheloid carcinoma, HepG2 (hepatocellular carcinoma), HEK 293 (embryonic kidney carcinoma), HT-29 (colorectal adenocarcinoma), HeLa (cervix carcinoma), PC-3 (prostate carcinoma) and B-LCL (EBV-transformed B-cells) were plated on petri-dishes or kept in suspension (B-LCL) and cultured in DMEM F0435 (w/o pyruvate), supplemented with 5% dialyzed fetal calf serum. All cells were treated independently with the above named antibiotics (0.1 - 1000 µM) and medium was exchanged on a daily basis. After 4 cell deviations of regular cell growth, in the presence of antibiotic, cell growth retarded and came to a halt after 6-8 days (see figure 5a-5l, top line in each panel). To see whether this side effect can be ameliorated by the addition of pyruvate and/or uridine (pyruvate: 1 mM sodium pyruvate; uridine: 0.2 mM uridine), cell lines growth was indistinguishable from untreated cells, demonstrating that the severe side effects of the antibiotics was completely abolished when growth media were supplemented with pyruvate and uridine (see figure 5a-5l, lower line in each panel).

### Example 2:

### Antibiofic effect is independent of co-administration of uridine and/or pyruvate

To prove whether or not the antibiotic effect of the drugs is rendered by the co-administration of uridine and/or sodium pyruvate, *E. coli* strain DH5□ or *B. subtilis* strain DSM 347) were plated on LB-agar plates or APT-plates that were used as is or supplemented with uridine (50 µg/ml), sodium pyruvate (1 mM) or uridine (50 µg/ml) + pyruvate (1 mM).

Paper test pads (Rotiolabo, 6mm, Roth, Karlsruhe) were soaked with 7.5 µl of antibiotic-solution (concentrations used: 0.5 mM CAP; 0.77 mM CAP; 1.2 mM CAP; 0.15 mM LIN; 0.25 mM LIN; 0,4 mM LIN; 0.15 mM TIG; 0.25 mM TIG; 0.4 mM TIG) and placed on top of the agar plates (CAP- and LIN-solutions on APT/B. *subtilis* plates, TIG-solutions on LB/E. *coli* plates). Petri dishes were incubated over night at 37°C and analyzed by measuring the size of the non-growth zone of bacteria surrounding the antibiotics soaked paper pads (see figure 3a, and 3b).

All dilutions of antibiotics applied presented an antibiotic effect independent from the co-administered substances (uridine and/or pyruvate). As background control, similar experiments were carried out without antibiotic solution under otherwise identical conditions. As expected, when pyruvate or uridine was administered solely or in combination *without* antibiotic, non-growths zones were obsolete. Thus, antibiotic effects of chloramphenicol, tigezycline or linezolid are solely based on the antibiotic compound and are not rendered by the co-administered compounds uridine and/or pyruvate.

### Example 3

### Antibiotic effect seen by combination of antibiotic compounds in the presence of uridine, pyruvate or uridine + pyruvate

To prove whether or not the antibiotic effect of the drugs is rendered by combining antibiotics and co-administering uridine and/or sodium pyruvate, *B. subtilis* strain DSM 347 was plated on APT-plates (*E. coli* strain DH5□ were grown on LB plates) that were used as is or supplemented with uridine (50 µg/ml), sodium pyruvate (1 mM) or uridine (50 µg/ml) + pyruvate (1 mM).

Paper test pads (Rotiolabo, 6mm, Roth, Karlsruhe) were soaked with 7.5 µl of antibiotic-solution (concentrations used: 0.5 mM CAP; 0.77 mM CAP; 1.2 mM CAP; 0.15 mM LIN; 0.25 mM LIN, 0.4 mM LIN; 0.15 mM TIG, 0.25 mM TIG; 0.4 mM TIG; 0.5 mM CAP + 0.15 mM LIN; 0.77 mM CAP + 0.25 mM LIN; 1.2 mM CAP + 0.4 mM LIN; 0.5 mM CAP + 0.15 mM LIN + 0.15 mM TIG; 0.77 mM CAP + 0.25 mM LIN + 0.25 mM TIG; 1.2 mM CAP + 0.4 mM LIN + 0.4 mM TIG; 0.5 mM CAP + 0.15 mM TIG; 0.77 mM CAP + 0.25 mM TIG; 1.2 mM CAP + 0.4 mM TIG) and placed on top of the agar plates (CAP- and LIN-solutions on APT/*B*. *subtilis* plates, TIG-solutions on LB/*E*. *coli* plates). Petri dishes were incubated over night at 37°C and analyzed by measuring the size of the non-growth zone of bacteria surrounding the antibiotics soaked paper pads (see figure 2a, and 2b).

All dilutions of antibiotics applied presented an antibiotic effect independent from the co-administered substances (uridine and/or pyruvate). As background control, similar experiments were carried out without antibiotic solution under otherwise identical conditions. As expected, when pyruvate or uridine was administered solely or in combination *without* antibiotic, non-growths zones were obsolete. Thus, antibiotic effects of chloramphenicol, tigezycline or linezolid are solely based on the antibiotic compound and are not rendered by the co-administered compounds uridine and/or pyruvate.

### Example 4

### Determination of IC₅₀ values of antibiotics in the presence of uridine and/or pyruvate

The half maximal inhibitory concentration (IC₅₀) is a measure of the effectiveness of a compound in inhibiting biological or biochemical function. This quantitative measure indicates, how much of a particular drug or compound is needed to inhibit a given biological process by half. In this case, the IC₅₀ value was determined for the side-effect of the antibiotic on human cells in the presence or absence of pyruvate and/or uridine. IC₅₀ values were determined utilizing resazurin (Cell-Quanti-Blue Cell Viability Assay Kit, Biotrend Chemikalien GmbH, Köln), a fluorescent dye indicating vital living or dead cells. For the experiment, 24-well culture dishes were seeded with a dilution of 143B.TK⁻ cells (10³ cells each). The culture medium was composed of DMEM FG0445 (containing sodium pyruvate, high-glucose, glutamine; at the discretion of the supplier) supplemented with 5% FCS and 0.5 % uridine. After an initial growth of two days, cells were washed in PBS. From here, cells were grown in DMEM F0435 (containg high-glucose, without pyruvate, without glutamine; at the discretion of the supplier) supplemented with 2 % glutamine and 5% FCS. Depending on the subsequent experiments, cell growth medium was further supplemented with:
1. w/o chloramphenicol, w/o pyruvate, w/o uridine
2. w chloramphenicol (50 nM - 300 µM), w/o pyruvate, w/o uridine
3. w chloramphenicol (50 nM - 300 µM), w/o pyruvate, w uridine (50 µg/ml)
4. w chloramphenicol (50 nM - 300 µM), w pyruvate (100 µg/ml), w/o uridine
5. w chloramphenicol (50 nM - 300 µM), w pyruvate (100 µg/ml), w uridine (50 µg/ml)

Growth medium was changed every 12 hours. The experiment was carried out for 10 days.

For analysis displayed in Figure 4, cell vitality (cell number) was measured utilizing resazurin. Measures of sets 2-5 were normalized by measure 1. Thus, when cell vitality under chloramphenicol treatment is similar to normal cell growth (measure 1), the ratio of (measure 2, 3, 4, 5) /(measure 1) expressed in % should stay close to 1. As can be seen in Figure 4, IC₅₀ of CAP treatment without uridine or pyruvate supplementation was approximately 3.2 µM. When cells were supplemented with uridine under otherwise identical conditions, the IC₅₀ of CAP shifted to 7.5 µM. When pyruvate or pyruvate + uridine were added to the growth medium, IC₅₀ of CAP was out of range (> 300 µM).

It can be easily seen from the displayed data that full supplementation (pyruvate + uridine) leads to an indistinguishable vitality of cells grown in CAP (ratio ∼ 1) when compared to cells grown in CAP without supplementation.

### Example 5:

### Ameliorating the side effects of antibiotics by applying alternative pyruvate or uridine sources

Side effects of antibiotics can be ameliorated by administration of pyruvate and uridine. This observation can be further substantiated by applying alternative pyruvate or uridine sources, as for example alanine and uridine monophosphate (UMP).
***Alternative pyruvate source:*** To challenge this hypothesis, cells devoid of mitochondrial DNA (rho zero cells) resembling cells that were treated with the antibiotics and have lost their mitochondrial encoded proteins, were cultured in the presence of uridine, alanine and uridine or pyruvate and uridine. In details HT 29 rho zero cells (devoid of mitochondrial DNA and thus mitochondrial encoded proteins) were seeded on petri-dishes and cultured in DMEM F0435 (w/o pyruvate), supplemented with 5% dialyzed fetal calf serum. Cells growth medium was supplemented with uridine (0.2 mM uridine) or alanine (1-10 mM alanine) and uridine (0.2 mM uridine) or pyruvate (1 mM sodium pyruvate) and uridine (0.2 mM uridine) (see figure 6a). While cells cultured without pyruvate or alanine (upper row) did not exhibit cell growth, cells supplemented with alanine (middle row) or pyruvate (lower row) displayed regular cell growth on uridine supplemented media. Thus, pyruvate can be substituted by alternative pyruvate sources to ameliorate side effects of antibiotics.
***Alternative uridine source:*** To challenge this hypothesis, cells devoid of mitochondrial DNA (rho zero cells) resembling cells that were treated with the antibiotics and have lost their mitochondrial encoded proteins, were cultured in the presence of UMP, alanine and UMP or pyruvate and UMP. In details HT 29 rho zero cells (devoid of mitochondrial DNA and thus mitochondrial encoded proteins) were seeded on petri-dishes and cultured in DMEM F0435 (w/o pyruvate), supplemented with 5% dialyzed fetal calf serum. Cells growth medium was supplemented with UMP (0.2 mM UMP) or alanine (1-10 mM alanine) and UMP (0.2 mM UMP) or pyruvate (1 mM sodium pyruvate) and UMP (0.2 mM UMP) (see figure 6b). While cells cultured without pyruvate or alanine on UMP supplemented growth media (upper row) did not exhibit cell growth, cells supplemented with alanine (middle row) or pyruvate (lower row) on UMP supplemented media displayed regular cell growth. Thus, UMP can be substituted by alternative pyrimidine source to ameliorate side effects of antibiotics.

Having described preferred embodiments of the invention, it is to be understood that the invention is not limited to the precise embodiments.

### Reference List

(1) Waksman, S. A. "What is an antibiotic or an antibiotic substance?" Mycotogia. 39.5 (1947): 565-69.
(2) Slama, T. G., et al. "A clinician's guide to the appropriate and accurate use of antibiotics: the Council for Appropriate and Rational Antibiotic Therapy (CARAT) criteria." Am.J.Med. 118 Suppl 7A (2005): 1S-6S.
(3) Falagas, M. E., A. P. Grammatikos, and A. Michalopoulos. "Potential of old-generation antibiotics to address current need for new antibiotics." Expert. Rev.Anti. Infect.Ther. 6.5 (2008): 593-600.
(4) McIntyre, J. and I. Choonara. "Drug toxicity in the neonate." Biol.Neonate 86.4 (2004): 218-21.
(5) Brunton, L. L. Chapter 46. Protein Synthesis Inhibitors and Miscellaneous Antibacterial Agents. Goodman & Gilman's The Pharmacological Basis of Therapeutics (11th ed.). 2006. McGraw-Hill.
(6) Yunis, A. A. "Chloramphenicol toxicity: 25 years of research." Am.J.Med. 87.3N (1989): 44N-8N.
(7) Shu, X. O., et al. "Chloramphenicol use and childhood leukaemia in Shanghai." Lancet 2.8565 (1987): 934-37.

## Claims

1. A composition comprising:
(i) a therapeutically effective amount of at least one antibiotic; and
(ii) a therapeutically effective amount of both an uridine compound and pyruvate compound
for use as a medicament, wherein
the uridine compound is uridine, uracil, uridine phosphate(s) or salt(s) thereof, UDP-glucose, cytidine-5'-monophosphate (CMP), cytidine-5'-diphosphate (CDP), CDP-glucose, orotic acid, orotidylate, acylated uridine or cytidine-diphosphocholine; and
the pyruvate compound is pyruvic acid or a salt thereof, creatine pyruvate, phosphoenolpyruvate, 2-phosphoglycerate, 3-phosphoglycerate, 1,3-biphosphoglycerate, glycerinaldehyde 3-phosphate, dihydroxyacetone phosphate, alanine, serine, cystein, glycine, tryptophan, asparagine, aspartate, methionine, malate or oxalacetate.

2. The composition for the use of claim 1, wherein the antibiotic is selected from the antibiotic classes of aminoglycosides, ansamycins, carbacephmes, cephalosporins, glycopeptides, macrolides, penicillins, polypeptides, quinolones, sulfonamides, tetracyclines, glycycyclines, oxazolidinones, amphenicols, pleuromutilins, lincosamides, streptogramins, steroid antibacterials, cyclopeptides, lipopeptides, and mixtures thereof.

3. The composition for the use of claim 2, wherein the antibiotic is selected from the group consisting of amikacin, gentamicin, kanamycin, neomycin, netilmicin, streptomycin, tobramycin, paromomycin, geldanamycin, herbimycin, loracarbef, ertapenem, doripenem, imipenem/cilastatin, meropenem, cefadroxil, duricef, cefazolin, cefalotin, cefalothin, cefalexin, cefaclor, cefamandole, cefoxitin, cefprozil, cefuroxime, cefixime, cefdinir, cefditoren, cefoperazone, cefotaxime, cefpodoxime, ceftazidime, ceftibuten, ceftizoxime, ceftriaxone, cefepime, ceftobiprole, teicoplanin, vancomycin, azithromycin, clarithromycin, dirithromycin, erythromycin, roxithromycin, troleandomycin, telithromycin, spectinomycin, aztreonam, amoxicillin, ampicillin, azlocillin, carbenicillin, cloxacillin, dicloxacillin, flucloxacillin, mezlocillinm, meticillin, nafcillin, oxacillin, penicillin, piperacillin, ticarcillin, ciprofloxacin, enoxacin, gatifloxacin, levofloxacin, lomefloxacin, fodaximicin, moxifloxacin, norfloxacin, ofloxacin, trovafloxacin, grepafloxacin, sparfloxacin, temafloxacin, mafenide, sulfonamidochrysoidine sulfacetamide, sulfadiazine, ulfamethizole, sulfanilamide, sulfasalizine, sulfisoxazole, trimethoprim, trimethroprim-sulfamethoxazole (co-trimoxazole) (TMP-SMX), doxycycline, minocycline, oxytetracycline, tetracycline, chloramphenicol, tigezycline, linezolid , clindamycin, lincomycin, ethambutol, myambutol, fosfomycin, fusidic acid, furazolidone, isoniazid, linezolid, metronidazole, mupirocin, nitrofurantoin, platensimycin, pyrazinamide, quinupristin/dalfopristin, rifampicin, thiamphenicol, tinidazole, dapsone, clofazimine dihydrostreptomycin, framycetin, ribostamycin, arbekacin, bekanamycin, dibekacin tobramycin, hygromycin B, sisomicin, isepamicin, verdamicin, astromicin, chlortetracycline clomocycline, demeclocycline, lymecycline, meclocycline, metacycline, penimepicycline, rolitetracycline, torezolid, eperezolid, posizolid, radezolid, azidamfenicol, florfenicol, retapamulin, tiamulin, valnemulin, flurithromycin, josamycin, midecamycin, miocamycin, oleandomycin, rokitamycin, spiramycin, tylosin, ketolides, cethromycin, solthromycin, pirlimycin, pristinamycin, virginiamycin, chloroquin, hydroxochloroquin, cefotaxim, polymyxin B, colistin, bacitracin, tyrotricin, gramicidin, daptomycin, fidaxomicin, and mixtures thereof.

4. The composition for the use of claim 3, wherein the antibiotic is chloramphenicol, linezolid or tigezycline.

5. The composition for the use of any of claims 1 to 3, wherein the pyruvate compound is methyl pyruvate, ethyl pyruvate or sodium salt of pyruvate.

6. The composition for the use of any of claims 1 to 5, wherein the uridine phosphate is uridine-5' monophosphate (UMP), uridine-5' diphosphate (UDP) or uridine-5' triphosphate (UTP).

7. The composition for the use of any of claims 1 to 6, wherein (i) the antibiotic and (ii) the uridine and pyruvate compounds are provided as separate entities.

8. A pharmaceutical composition comprising
(i) a therapeutically effective amount of at least one antibiotic; and
(ii) a therapeutically effective amount of both an uridine compound and a pyruvate compound
for use in preventing or minimizing adverse side effects in the antibiotic therapy of bacterial infections in a subject, wherein
the uridine compound is uridine, uracil, uridine phosphate(s) or salt(s) thereof, UDP-glucose, cytidine-5' monophosphate (CMP), cytidine-5' diphosphate (CDP), CDP-glucose, orotic acid, orotidylate, acylated uridin or cytidine-diphosphocholine; and
the pyruvate compound is pyruvic acid or a salt thereof, creatine pyruvate, phosphoenolpyruvate, 2-phosphoglycerate, 3-phosphoglycerate, 1,3-biphosphoglycerate, glycerinaldehyde 3-phosphate, dihydroxyacetone phosphate, alanine, serine, cystein, glycine, tryptophan, asparagine, aspartate, methionine, malate or oxalacetate.

9. The pharmaceutical composition for the use of claim 8, wherein the adverse side effects in antibiotic therapy to be prevented or minimized are rash, diarrhea, abdominal pain, nausea/vomiting, drug fever, hypersensitivity (allergic) reactions, serum sickness, vaginal candidiasis, renal (kidney) toxicity, ototoxicity (hearing loss), dizziness, nystagmus, headache, liver toxicity, eosinophilia (elevated white blood cells), anorexia, hemolytic anemia, peripheral neuropathy, dizziness, reddish-orange body fluids, flushing, hypotension, itching, phlebitis, taste, taste alterations, photosensitivity , tooth discoloration in children, lethargy, insomnia, photosensitivity, pseudomembranous colitis, jaundice and/or metallic taste.

10. The pharmaceutical composition for the use of claim 8 or 9, wherein (i) the antibiotic and (ii) the uridine and pyruvate compounds are in a unit dosage form to be administered simultaneously.

11. The pharmaceutical composition for the use of any of claims 8 to 10, wherein the pyruvate compound is methyl pyruvate, ethyl pyruvate or sodium salt of pyruvate.

12. The pharmaceutical composition of any of claims 8 to 11, wherein the uridine phosphate is uridine-5' monophosphate (UMP) or a salt thereof, uridine-5' diphosphate (UDP) or uridine-5' triphosphate (UTP).

## Patentansprüche

1. Zusammensetzung, umfassend:
i) eine therapeutisch wirksame Menge von mindestens einem Antibiotikum; und
ii) eine therapeutisch wirksame Menge sowohl einer Uridinverbindung als auch einer Pyruvatverbindung
zur Verwendung als Arzneimittel, wobei
die Uridinverbindung Uridin, Uracil, Uridinphosphat(e) oder Salz(e) davon, UDP-Glucose, Cytidin-5'-monophosphat (CMP), Cytidin-5'-diphosphat (CDP), CDP-Glucose, Orotsäure, Orotidylat, acyliertes Uridin oder Cytidin-Diphosphocholin ist; und
die Pyruvatverbindung Brenztraubensäure oder ein Salz davon, Creatinpyruvat, Phosphoenolpyruvat, 2-Phosphoglycerat, 3-Phosphoglycerat, 1,3-Biphosphoglycerat, Glycerinaldehyd 3-Phosphat, Dihydroxyacetonphosphat, Alanin, Serin, Cystein, Glycin, Tryptophan, Asparagin, Aspartat, Methionin, Malat oder Oxalacetat ist.

2. Zusammensetzung zur Verwendung nach Anspruch 1, wobei das Antibiotikum ausgewählt ist aus den Antibiotika-Klassen von Aminoglykosiden, Ansamycinen,Carbacephmen, Cephalosporinen, Glycopeptiden, Makroliden, Penicillinen, Polypeptiden, Chinolonen, Sulfonamiden, Tetracyclinen, Glycycyclinen, Oxazolidinonen, Amphenicolen, Pleuromutilinen, Lincosamiden, Streptograminen, Steroid-Antibiotika, Cyclopeptide, Lipopeptide, und Mischungen davon.

3. Zusammensetzung zur Verwendung nach Anspruch 2, wobei das Antibiotikum ausgewählt ist aus der Gruppe bestehend aus Amicacin, Gentamicin, Kanamycin, Neomycin, Netilmicin, Streptomycin, Tobramycin, Paromomycin, Geldanamycin, Herbimycin, Loracarbef, Ertapenem, Doripenem, Imipenem/Cilastatin, Meropenem, Cefadroxil, Duricef, Cefazolin, Cefalotin, Cefalothin, Cefalexin, Cefaclor, Cefamandol, Cefoxitin, Cefprozil, Cefuroxim, Cefixim, Cefdinir, Cefditoren, Cefoperazon, Cefotaxim, Cefpodoxim, Ceftazidim, Ceftibuten, Ceftizoxim, Ceftriaxon, Cefepim, Ceftobiprol, Teicoplanin, Vancomycin, Azithromycin, Clarithromycin, Dirithromycin, Erythromycin, Roxithromycin, Troleandomyzin, Telithromycin, Spectinomycin, Aztreonam, Amoxicillin, Ampicillin, Azlocillin, Carbenicillin, Cloxacillin, Dicloxacillin, Flucloxacillin, Mezlocillinm, Methicillin, Nafcillin, Oxacillin, Penicillin, Piperacillin, Ticarcillin, Ciprofloxacin, Enoxacin, Gatifloxacin, Levofloxacin, Lomefloxacin, Fodaximicin, Moxifloxacin, Norfloxacin, Ofloxacin, Trovafloxacin, Grepafloxacin, Sparfloxacin, Temafloxacin, Mafenid, Sulfonamidochrysoidin Sulfacetamid, Sulfadiazin, Sulfamethizol, Sulfanilamid, Sulfasalizine, Sulfisoxazol, Trimethoprim, Trimethoprim-Sulfamethoxazol (Co-Trimoxazol) (TMP-SMX), Doxycyclin, Minocyclin, Oxytetracyclin, Tetracyclin, Chloramphenicol, Tigezyclin, Linezolid, Clindamycin, Lincomycin, Ethambutol, Myambutol, Fosfomycin, Fusidinsäure, Furazolidon, Isoniazid, Linezolid, Metronidazol, Mupirocin, Nitrofurantoin, Platensimycin, Pyrazinamid, Quinupristin/Dalfopristin, Rifampicin, Thiamphenicol, Tinidazol, Dapson, Clofazimin-Dihydrostreptomycin, Framycetin, Ribostamycin, Arbekacin, Bekanamycin, Dibekacin, Tobramycin, Hygromycin B, Sisomicin, Isepamicin, Verdamicin, Astromicin, Chlortetracyclin, Clomocyclin, Demeclocyclin, Lymecyclin, Meclocyclin, Metacyclin, Penimepicyclin, Rolitetracyclin, Torezolid, Eperezolid, Posizolid, Radezolid, Azidamfenicol, Florfenicol, Retapamulin, Tiamulin, Valnemulin, Flurithromycin, Josamycin, Midecamycin, Miocamycin, Oleandomycin, Rokitamycin, Spiramycin, Tylosin, Ketoliden, Cethromycin, Solthromycin, Pirlimycin, Pristinamycin, Virginiamycin, Chlorochquin, Hydroxochlorochquin, Cefotaxim, Polymyxin B, Colistin, Bacitracin, Tyrotricin, Gramicidin, Daptomycin, Fidaxomicin und Mischungen davon .

4. Zusammensetzung zur Verwendung nach Anspruch 3, wobei das Antibiotikum Chloramphenicol, Linezolid oder Tigezyclin ist.

5. Zusammensetzung zur Verwendung nach einem der Ansprüche 1 bis 3, wobei die Pyruvat-Verbindung Methylpyruvat, Ethylpyruvat oder ein Natriumsalz von Pyruvat ist.

6. Zusammensetzung nach einem der Ansprüche 1 bis 5, wobei das Uridinphosphat Uridin-5'-monophosphat (UMP), Uridin-5'-diphosphat (UDP) oder Uridin-5'-triphosphat (UTP) ist.

7. Zusammensetzung zur Verwendung nach einem der Ansprüche 1 bis 6, wobei (i) das Antibiotikum und (ii) die Uridin- und Pyruvat-Verbindungen als getrennte Einheiten bereitgestellt werden.

8. Pharmazeutische Zusammensetzung, umfassend
i) eine therapeutisch wirksame Menge von mindestens einem Antibiotikum; und
ii) eine therapeutisch wirksame Menge sowohl einer Uridin-Verbindung als auch einer Pyruvat-Verbindung
zur Verwendung bei der Verhinderung oder Minimierung von nachteiligen Nebenwirkungen in der antibiotischen Therapie von bakteriellen Infektionen in einem Subjekt, wobei
die Uridin-Verbindung Uridin, Uracil, Uridinphosphat(e) oder Salz(e) davon, UDP-Glucose, Cytidin-5'-monophosphat (CMP), Cytidin-5'-diphosphat (CDP), CDP-Glucose, Orotsäure , Orotidylat, acyliertes Uridin oder Cytidin-Diphosphocholin ist; und
die Pyruvatverbindung Brenztraubensäure oder ein Salz davon, Creatinpyruvat, Phosphoenolpyruvat, 2-Phosphoglycerat, 3-Phosphoglycerat, 1,3-Biphosphoglycerat, Glycerinaldehyd 3-Phosphat, Dihydroxyacetonphosphat, Alanin, Serin, Cystein, Glycin, Tryptophan, Asparagin, Aspartat, Methionin, Malat oder Oxalacetat ist.

9. Pharmazeutische Zusammensetzung für die Verwendung nach Anspruch 8, wobei die nachteiligen Nebenwirkungen in der Antibiotika-Therapie, die verhindert oder minimiert werden, Hautausschlag, Durchfall, Bauchschmerzen, Übelkeit/Erbrechen, Drogenfieber, Überempfindlichkeits (allergische) -Reaktionen, Serumkrankheit, vaginale Candidiasis, renale (Nieren) Toxizität, Ototoxizität (Hörverlust), Schwindel, Nystagmus, Kopfschmerzen, Lebertoxizität, Eosinophilie (erhöhte weiße Blutkörperchen), Anorexie, hämolytische Anämie, periphere Neuropathie, Schwindel, rötlich-orange Körperflüssigkeiten, Erröten, Hypotonie, Juckreiz, Phlebitis, Geschmacksveränderungen, Lichtempfindlichkeit, Zahnverfärbung bei Kindern, Lethargie, Schlaflosigkeit, Lichtempfindlichkeit, pseudomembranöse Kolitis, Gelbsucht und/oder metallischer Geschmack sind.

10. Pharmazeutische Zusammensetzung zur Verwendung nach Anspruch 8 oder 9, wobei (i) das Antibiotikum und (ii) die Uridin- und Pyruvat-Verbindungen in einer gleichzeitig zu verabreichenden Einheitsdosierungsform vorliegen.

11. Pharmazeutische Zusammensetzung zur Verwendung nach Anspruch 8 oder 9, wobei die Pyruvat-Verbindung, Methylpyruvat, Ethylpyruvat oder ein Natriumsalz von Pyruvat ist.

12. Pharmazeutische Zusammensetzung nach einem der Ansprüche 8 bis 11, wobei das Uridinphosphat Uridin-5'-monophosphat (UMP) oder ein Salz davon, Uridin-5'-diphosphat (UDP) oder Uridin-5'-triphosphat (UTP) ist.

## Revendications

1. Composition comprenant:
(i) une quantité thérapeutiquement efficace d'au moins un antibiotique; et
(ii) une quantité thérapeutiquement efficace tant d'un composé d'uridine que d'un composé de pyruvate
destinée à être utilisée comme médicament, dans laquelle
le composé d'uridine est l'uridine, l'uracile, le ou les phosphates d'uridine ou leur ou leurs sels, l'UDP-glucose, la cytidine-5-monophosphate (CMP), la cytidine-5-diphosphate (CDP), le CDP-glucose, l'acide orotique, l'orotidylate, l'uridine acylée ou la cytidine-diphosphocholine; et
le composé de pyruvate est l'acide pyruvique ou un sel de ce dernier, le pyruvate de créatine, le phosphoénolpyruvate, le 2-phosphoglycérate, le 3-phosphoglycérate, le 1,3-biphosphoglycérate, le glycérinaldéhyde-3-phosphate, la dihydroxyacétone phosphate, l'alanine, la sérine, la cystéine, la glycine, le tryptophane, l'asparagine, l'aspartate, la méthionine, le malate ou l'oxalacétate.

2. Composition destinée à être utilisée selon la revendication 1, dans laquelle l'antibiotique est choisi parmi les classes d'antibiotiques des aminoglycosides, des ansamycines, des carbacéphèmes, des céphalosporines, des glycopeptides, des macrolides, des pénicillines, des polypeptides, des quinolones, des sulfonamides, des tétracyclines, des glycycyclines, des oxazolidinones, des amphénicols, des pleuromutilines, des lincosamides, des streptogramines, des antibactériens stéroïdiens, des cyclopeptides, des lipopeptides, et des mélanges de ces derniers.

3. Composition destinée à être utilisée selon la revendication 2, dans laquelle l'antibiotique est choisi dans le groupe composé de l'amikacine, la gentamicine, la kanamycine, la néomycine, la nétilmicine, la streptomycine, la tobramycine, la paromomycine, la geldanamycine, l'herbimycine, le loracarbef, l'ertapénem, le doripénem, l'imipénem/la cilastatine, le méropénem, le céfadroxil, le duricef, la céfazoline, la céfalotine, la céphalothine, la céfalexine, le cefaclor, le céfamandole, la céfoxitine, le cefprox, la céfuroxime, la céfixime, le cefdinir, le cefditoren, la céfopérazone, la céfotaxime, la cefpodoxime, le ceftazidime, le ceftibutène, le ceftizoxime, la ceftriaxone, le ceftibime, le ceftobiprole, la téicoplanine, la vancomycine, l'azithromycine, la clarithromycine, la dirithromycine, l'érythromycine, la roxithromycine, la troléandomycine, la télithromycine, la spectinomycine, l'aztréonam, l'amoxicilline, l'ampicilline, l'azlocilline, la carbénicilline, la cloxacilline, la dicloxacilline, la flucloxacilline, la mezlocilline, la méticilline, la nafcilline, l'oxacilline, la pénicilline, la pipéracilline, la ticarcilline, la ciprofloxacine, l'énoxacine, la gatifloxacine, la lévofloxacine, la loméfloxacine, la fodaximicine, la moxifloxacine, la norfloxacine, l'ofloxacine, la trovafloxacine, la grépafloxacine, la sparfloxacine, la témafloxacine, le mafénide, la sulfonamidochrysoîdine sulfacétamide, la sulfadiazine, l'ulfaméthizole, le sulfanilamide, la sulfasalizine, le sulfisoxazole, le triméthoprime, le triméthodromesulfaméthoxazole (co-trimoxazole) (TMP-SMX), la doxycycline, la minocycline, l'oxytétracycline, la tétracycline, le chloramphénicol, la tigécycline, le linézolide, la clindamycine, la lincomycine, l'éthambutol, le myambutol, la fosfomycine, l'acide fusidique, la furazolidone, l'isoniazide, le linézolide, le métronidazole, la mupirocine, la nitrofurantoïne, la platensimycine, la pyrazinamide, la quinupristine/dalfopristine, la rifampicine, le thiamphénicol, le tinidazole, la dapsone, la clofazimine la dihydrostreptomycine, la framycétine, la ribostamycine, l'arbékacine, la békanamycine, la dibékacine tobramycine, l'hygromycine B, la sisomicine, l'isépamicine, la verdamicine, l'astromicine, la chlortétracycline, la clomocycline, la démécyclocycline, la lymécycycline, la méclocycline, la métacycline, la pénimépicycline, la rolitétracycline, le torézolide, l'épélose, le posizolide, le radézolide, l'azidamfénicol, le florfénicol, la rétapamuline, la tiamuline, la valnémuline, la flurithromycine, la josamycine, la midécamycine, la miocamycine, l'oléandomycine, la rokitamycine, la spiramycine, la tylosine, les cétolides, la céthromycine, la solthromycine, la pyrlimycine, la pristinamycine, la virginiamycine, la chloroquine, l'hydroxochloroquine, le céfotaxime, la polymyxine B, la colistine, la bacitracine, la tyrotricine, la gramicidine, la daptomycine, la fidaxomicine et les mélanges de ces derniers.

4. Composition destinée à être utilisée selon la revendication 3, dans laquelle l'antibiotique est le chloramphénicol, le linézolide ou la tigécycline.

5. Composition destinée à être utilisée selon l'une quelconque des revendications 1 à 3, dans laquelle le composé de pyruvate est le pyruvate de méthyle, le pyruvate d'éthyle ou le sel de sodium de pyruvate.

6. Composition destinée à être utilisée selon l'une quelconque des revendications 1 à 5, dans laquelle le phosphate d'uridine est l'uridine-5-monophosphate (UMP), l'uridine-5-diphosphate (UDP) ou l'uridine-5-triphosphate (UTP).

7. Composition destinée à être utilisée selon l'une quelconque des revendications 1 à 6, dans laquelle (i) l'antibiotique et (ii) les composés d'uridine et de pyruvate sont fournis comme entités séparées.

8. Composition pharmaceutique comprenant
(i) une quantité thérapeutiquement efficace d'au moins un antibiotique; et
(ii) une quantité thérapeutiquement efficace tant d'un composé d'uridine que d'un composé de pyruvate
destinée à être utilisée dans la prévention ou la minimisation des effets secondaires indésirables dans la thérapie antibiotique d'infections bactériennes chez une personne, où
le composé d'uridine est l'uridine, l'uracile, le ou les phosphates d'uridine ou leur ou leurs sels, l'UDP-glucose, la cytidine-5-monophosphate (CMP), la cytidine-5-diphosphate (CDP), le CDP-glucose, l'acide orotique, l'orotidylate, l'uridine acylée ou la cytidine-diphosphocholine; et
le composé de pyruvate est l'acide pyruvique ou un sel de ce dernier, le pyruvate de créatine, le phosphoénolpyruvate, le 2-phosphoglycérate, le 3-phosphoglycérate, le 1,3-biphosphoglycérate, le glycérinaldéhyde-3-phosphate, la dihydroxyacétone phosphate, l'alanine, la sérine, la cystéine, la glycine, le tryptophane, l'asparagine, l'aspartate, la méthionine, le malate ou l'oxalacétate.

9. Composition pharmaceutique destinée à être utilisée selon la revendication 8, dans laquelle les effets secondaires indésirables dans la thérapie antibiotique à prévenir ou minimiser sont l'éruption cutanée, la diarrhée, les douleurs abdominales, les nausées/vomissements, la fièvre médicamenteuse, les réactions (allergiques) d'hypersensibilité, la maladie du sérum, la candidose vaginale, la toxicité rénale (reins), l'ototoxicité (perte auditive), les vertiges, le nystagmus, les céphalées, la toxicité hépatique, éosinophilie (quantité élevée de globules blancs), l'anorexie, l'anémie hémolytique, la neuropathie périphérique, les vertiges, les fluides corporels rouge-orange, le rougissement, l'hypotension, les démangeaisons, la phlébite, le goût, les altérations du goût, la photosensibilité, la décoloration des dents chez les enfants, la léthargie, l'insomnie, la photosensibilité, la colite pseudo-membraneuse, la jaunisse et/ou le goût métallique.

10. Composition pharmaceutique destinée à être utilisée selon la revendication 8 ou 9, dans laquelle (i) l'antibiotique et (ii) les composés d'uridine et de pyruvate se présentent sous forme de dosage unitaire à administrer simultanément.

11. Composition pharmaceutique destinée à être utilisée selon l'une quelconque des revendications 8 à 10, dans laquelle le composé pyruvate est le pyruvate de méthyle, le pyruvate d'éthyle ou le sel de sodium de pyruvate.

12. Composition pharmaceutique selon l'une quelconque des revendications 8 à 11, dans laquelle le phosphate d'uridine est l'uridine-5-monophosphate (UMP) ou un sel de ce dernier, l'uridine-5-diphosphate (UDP) ou l'uridine-5-triphosphate (UTP).
